# EUROPÄISCHE PATENTSCHRIFT

(11) **EP 2 385 835 B1**
(45) Veröffentlichungstag und Bekanntmachung des Hinweises auf die Patenterteilung: **29.07.2015**
(21) Anmeldenummer: 10701905.1
(22) Anmeldetag: 06.01.2010
(51) Int. Cl.: A61K 33/00, A61P 27/02, A61P 27/06, A61P 27/12, A61P 31/12, A61P 31/14, A61P 31/16, A61P 31/18, A61P 31/20, A61P 31/22

(54) **VERWENDUNG VON DEUTERIUMOXID ZUR BEHANDLUNG VIRALER ERKRANKUNGEN DES AUGES**
USE OF DEUTERIUM OXIDE FOR TREATING VIRAL DISEASES OF THE EYE
UTILISATION D'OXYDE DE DEUTÉRIUM POUR LE TRAITEMENT D'AFFECTIONS VIRALES DE L'OEIL

(30) Priorität: 07.01.2009 DE 102009003942
(43) Veröffentlichungstag der Anmeldung: 16.11.2011
(73) Patentinhaber: D2 Bioscience Group Ltd, Hamilton HMLX (BM)
(72) Erfinder: BAYERL, Thomas, London SW7 3LR (GB)
(74) Vertreter: Ettmayr, Andreas
(86) Internationale Anmeldenummer: PCT/IB2010/000029
(87) Internationale Veröffentlichungsnummer: WO 2010/079421

(56) Entgegenhaltungen:
- EP-A1- 2 110 132
- WO-A2-2005/063281
- DE-A1-102007 031 397
- US-A1- 2006 159 771
- MUELLER-BREITKREUTZ KONSTANZE ET AL: "Inactivation of viruses by chemically and photochemically generated singlet molecular oxygen", JOURNAL OF PHOTOCHEMISTRY AND PHOTOBIOLOGY B BIOLOGY, vol. 30, no. 1, 1995, pages 63-70, ISSN: 1011-1344
- COLIN JOSEPH: "Ganciclovir ophthalmic gel, 0.15%: a valuable tool for treating ocular herpes.", CLINICAL OPHTHALMOLOGY (AUCKLAND, N.Z.) DEC 2007, vol. 1, no. 4, December 2007 (2007-12), pages 441-453, ISSN: 1177-5467

## Beschreibung

Die vorliegende Erfindung betrifft die Verwendung von Deuteriumdioxid (D20) zur Prophylaxe und/oder Therapie von Virus-basierten Erkrankungen des Auges.

US2006/159771 offenbart die Kombination von Glykosaminoglykan und Deuteriumoxid zur Behandlung von Netzhautablösung, wobei die Kombination topisch, in flüssiger Form, verabreicht wird.

WO2005/063281 offenbart die Verwendung von chemischen Chaperonen wie z. B. Deuteriumoxid, das die Freisetzung und die Replikation von verschiedenen Viren hemmt: Influenzavirus-A, Retroviren, Lentiviren, humanes Immundefizienzvirus, Erreger von Infektionserkrankungen, wie Pocken, Masern, Polio und Herpesvirusinfektionen, AIDS, Grippe.

DE102007031397 offenbart Deuteriumoxid zur Prophylaxe und/oder Therapie von Herpesvirus-basierten und Warzenvirus-basierten Erkrankungen, d.h. Keratoconjunctivitis herpetica.

EP2110132 offenbart Deuteriumoxid zur Behandlung von allergischer Konjunktivitis. MUELLER-BREITKREUTZ KONSTANZE ET AL: "Inactivation of viruses by chemically and photochemically generated singlet molecular oxygen", offenbart Deuteriumoxid für die Inaktivierung von HSV-1 und SHV-1. Gegenstände, die nicht durch den Schutzumfang der Ansprüche umfasst sind, sind nicht Teil der vorliegenden beanspruchten Erfindung.

Durch Viren hervorgerufene Erkrankungen (Virusinfektionen) sind weltweit verbreitet und stellen, insbesondere aufgrund der hohen Variabilität, Anpassungsfähigkeit und Mutationsrate von Viren, ein ernsthaftes Problem in der Medizin dar. Viren sind kleine Partikel von ca. 15 bis 400 nm Durchmesser, die nicht in der Lage sind, sich selbst zu replizieren, sondern hierfür eine Wirtszelle benötigen. Ihrer Wirtsspezifität entsprechend werden Viren unterschieden, die Tiere (Invertebraten und Vertebraten), Pflanzen, Bakterien oder Algen, Pilze und Protozoen infizieren. Virusinfektionen zeichnen sich allgemein durch eine hohe Vermehrungsrate der Viruspartikel in den befallenen Wirtszellen aus, welche mit einem Exponential- oder Potenzgesetz beschrieben werden kann. Der Vermehrungszyklus von Viren erfolgt über die Injektion ihrer Nukleinsäure (Virus-RNA oder Virus-DNA) in die Wirtszelle, in welcher die Replikation der Nukleinsäure durch Ausnutzung des Replikationsapparates der Wirtszelle erfolgt. Hierbei unterscheidet man den lytischen und den lysogenen Zyklus. Bei dem lytischen Zyklus (aktive Infektion), erfolgt nach der Injektion der Nukleinsäure die Replikation der Virus-Nukleinsäure im Zellkern der Wirtszelle, ein Zusammenbau der neuen Viruspartikel im Cytoplasma, worauf folgend die Wirtszelle schließlich lysiert (zerstört) wird und die Viren freigesetzt werden. Die so freigesetzten Viren infizieren weitere Wirtszellen. Bei dem lysogenen Zyklus wird die Nukleinsäure des Virus in das Genom der Wirtszelle integriert, wo sie zunächst verbleibt, ohne die Wirtszelle zu zerstören. Durch äußere Einflüsse (z.B. UV-Strahlung, Zugabe von mutagenen Substanzen) kann dieser lysogene Zyklus in einen vorangehend beschriebenen lytischen Zyklus übergehen. Im Fall von RNA-Viren ist nach deren Infektion eine Umschreibung der RNA in DNA notwendig, damit eine Replikation durch die Wirtszelle erfolgen kann. Dieser Vorgang erfolgt über die reverse Transkriptase, einem Enzym, das durch Virusgene kodiert wird und zunächst in der Wirtszelle synthetisiert werden muss, um die Virus-RNA in Virus-DNA umzuschreiben, die dann wiederum von der DNA-Polymerase der Wirtszelle repliziert wird. Viren sind in der Lage, ein breites Spektrum an Zellen, Organen und Wirten zu infizieren. Jede Virusart infiziert spezifisch bevorzugte Zellen, wie beispielsweise Zellen des Magens, Darms, der Haut und des Auges. Dies führt zu zahlreichen sogenannten Virus-basierten Erkrankungen.

Virus-basierte Erkrankungen des Auges stellen hierbei ein schwerwiegendes Problem in der Pathologie, insbesondere des Menschens, dar.

Viren, die solche Virus-basierten Erkrankungen des Auges hervorrufen, umfassen insbesondere Herpes-Viren, wie Herpes simplex Virus 1, Herpes simplex Virus 2, Epstein-Barr-Virus, Varizella Zoster Virus und Cytomeglovirus, Adenoviren, Influenzaviren, Parainfluenzaviren, Picornaviren, wie Rhinoviren, Coxackievirus A, Coxsackievirus B und Echoviren, Molluscum contagiosum Virus (Poxvirus), Vacciniaviren, Mumpsviren, Masernviren, Rötelviren, Polioviren, Tollwutviren, humane Immundefizienzviren und das Rifttal-Fieber-Virus, sie sind jedoch nicht auf diese beschränkt.

Die Übertragung der Viren erfolgt zumeist durch Tröpfchen- und Schmierinfektionen. Die Viren infizieren in der Regel zunächst Hautzellen, wie Epithelzellen, Schleim- und Schleimhautzellen, des Auges gefolgt von einer starken Vermehrung des Virus in der Wirtszelle und einem Absterben der infizierten Wirtszelle. Der Wirt reagiert mit einer Immunantwort, die zu verschiedenen symptomatischen Krankheitsbildern führt.

Zu Virus-basierten Erkrankungen des Auges zählen beispielsweise Keratokonjunktividen oder Konjunktividen, insbesondere Keratokonjunktivitis epidemica, Keratokonjunktivitis sicca, hemorrharische Konjunktivitis, follikuläre Konjunktivitis, Pharyngokonjunktivalfieber, Blepharitis, Blepharokonjunktivitis, Herpes simplex-Blepharitis, Herpes simplex-Keratitis, Keratitis dendritica, Keratitis interstitialis, Edotheliitis/Keratitis disciformis, (Varizella) Zoster opthalamicus, Ophthalmia neonatorum (Neugeborenenkonjunktivitis), Molluscum contagisum, Canaliculitis, akute Dakryoadentitis, Dakryocystose, akute retinale Nekrose, Episkleritis, Skleritis, Chorioditis, Chorioretinits, Iritis, Iridocyclitis, Retinitis, Rhinokonjunktivitis sowie Uveitiden, wie Uveitis anterior, Uveitis intermediate, Uveitis posterior, Panuveitis, und Uveoretinitis. Weiterhin können Katarakt, Augenmuskelparese und kogenitales Glaukom hervorgerufen werden. Virus-basierte Erkrankungen des Auges treten häufig sekundär zu Virus-Infektionen des Respirationstraktes, z.B. der Influenza und der Rhinitis, auf.

Die Symptomatik und somit das Krankheitsbild, das durch Virusinfektionen des Auges herbeigeführt wird, ist bei den meisten Viren, mit Ausnahme der Herpes-Viren, ähnlich. Einige der Virus-basierten Erkrankungen des Auges können von verschiedenen Viren hervorgerufen werden, einige werden bevorzugt von einem spezifischen Virustyp (auch Virusart) hervorgerufen. Die Keratokonjunktivitis z.B. wird in den meisten Fällen von Andenoviren hervorgerufen, kann aber auch durch andere Viren, wie Zytomeglieviren, hervorgerufen werden. Neben dem Virus, das die Infektion hervorruft, hängt die Symptomatik von der Schwere der Infektion und der Immunabwehr des Wirtes ab.

Sehr häufig werden Virus-basierte Erkrankungen des Auges durch Infektionen durch alpha-Herpesviren (HSV-1, Varizella Zoster) und Adenoviren hervorgerufen. Nachfolgend werden einige solcher spezifischen Viren, deren Infektionsweg und pathologisches Erscheinungsbild beispielhaft näher erläutert:

### Infektionen durch Herpesviridae

### 1. Infektionen durch alpha-Herpesviren

Herpesviren sind bei Wirbeltieren, insbesondere bei Säugetieren, und vor allem bei Mensch, Pferd, Schwein, Rind, Ziege, Schaf, Katze und Hund weit verbreitet. Humanpathogene Herpesviren (HHV) werden in Alpha-, Beta- und Gamma-Herpesviren (HHV-1 bis HHV-8) unterschieden, wobei den Alpha- und Gammaviren auch Viren angehören, die Tiere infizieren können, wie beispielsweise Pferd (Equines Herpesvirus), Rind (bovines Herpesvirus), Schwein (porcines Herpesvirus), Katze (felines Herpesvirus), Hund (canines Herpesvirus) und Huhn (Hühner-Herpesvirus 1).

Unter den humanpathogenen, d.h. den Menschen befallenden, Herpesviren sind insbesondere die Alpha-Herpesviren von großer Bedeutung. Zu den Alpha-Herpesviren werden das Herpes simplex Virus 1 (HSV-1), Herpes simplex Virus 2 (HSV-2) und Varizella Zoster Virus (VZV) gezählt.

Humane alpha-Herpesviren vermehren sich zunächst nach dem lytischen Zyklus in den Häuten des Auges, insbesondere den Epithelzellen, sowie auch in Mund- und Nasenschleimhaut (Primärinfektionen) und rufen in der Regel eine lokale Schädigung hervor, und zwar in Form einer Läsion, meistens eines Bläschens. Bei der Vermehrung freigesetzte Viren infizieren weiterhin bestimmte Nervenzellen (Neuronen), indem sie sich an Rezeptoren der Nervenenden anheften, die zu den Nervenknoten des Gesichtsnervs (Trigeminus) führen. Die Virus-DNA dringt in ein Axon ein, wird in das Cytoplasma der Nevenzellen und schließlich in deren Zellkern befördert. Dort erfolgt der Einbau der Virus-DNA in das Genom der Nervenzelle, dies führt zu einem Ruhezustand (Latenz), in dem nur wenige virale Gene exprimiert werden (lysogener Zyklus). Verschiedene äußere Reize können zu einer erneuten Aktivierung des Virus führen, welches letztendlich die Zerstörung (Lyse) der Nervenzelle zur Folge hat. Die im Rahmen einer Aktivierung entstehenden Virusnachkommen werden zunächst durch das Axon über den Trigeminus an die Stelle bzw. in das Gebiet der ursprünglichen Infektion transportiert und an den Nervenendigungen freigesetzt. Die Viren infizieren dort wiederum Epithelzellen und Endothelzellen sowie in fortgeschrittenen Fällen bei Befall des Auges auch das Hornhautstroma (Sekundärinfektionen).

Die oben beschriebenen primären HSV-1-Infektionen des Auges betreffen vorwiegend die Lidhaut, die Bindehaut und die oberen Schichten der Hornhaut. Darüber hinaus können die Häute und Schleimhäute der Tränensäcke, der Tränenkanälchen und der Tränendrüsen durch das Virus infiziert werden. Die beschriebenen Sekundärinfektionen mit HSV-1 betreffen sowohl oberflächliche als auch tiefere Hautschichten, z.B. wie tiefe Schichten der Hornhaut, die Aderhaut, die Lederhaut und weitere Bereiche der Uvea. Insbesondere werden die Epithelzellen dieser vorstehend beschriebenen Häute durch das Virus infiziert.

HSV-1 Infektionen des Auges können zu verschiedenen Krankheitsbildern, insbesondere Dakryozystose, Kanaliculitis, Dakryadenitis, Herpes-Simplex-Blepharitis, Herpes simplex-Keratitis (Keratitis dendritica, die Keratitis interstitialis, Edotheliitis), Keratitis disciformis, Uveitis, akute nekrotisierende Retinitis, Skleritis und follikuläre Konjunktivitis führen. Die Krankheitsbilder der sekundären Infektionen sind zumeist schwerwiegender als die der Primärinfektion.

### 2. Infektionen durch Varizella zoster Viren

Infektionen des Auges mit Varizella zoster Viren erfolgen sekundär zu Windpockenerkrankungen. Varizella zoster Viren rufen ebenfralls die Erkrankung Zoster opthalamicus (Gesichtsrose) hervor. Diese Erkrankung beginnt meistens mit Brennen und Schmerzen in einer Gesichts- und Stirnhälfte. Nach einigen Tagen kommt es zur Rötung der Haut und Bläschenbildung. Im Bereich des Auges kann es durch die Virusinfektion zu Lidschwellungen durch Bläschenbildung auf den Augenlidern, Bindehautentzündungen und Hornhautentzündungen kommen.

Etwa 0,5 % aller Augenkrankheiten sollen durch Herpesviridae bedingt sein. Über 50 % aller Betroffenen erleiden nach der Ersterkrankung weitere Rückfälle und somit schwerwiegendere Sekundärinfektionen.

### Infektion durch Adenoviren

Adenoviren sind DNA-Viren, die sowohl Tiere als auch Menschen infizieren. Von insgesamt 19 Arten sind 6 humanpathogen (Humanes Adenovirus A-F). Humanpathogene Typen, die Virus-basierte Erkrankunden des Auges hervorrufen sind insbesondere die Stämme 1,2,3,4,5,6,7,8,9,10,11,13,14,15,19 und 29.

Adenoviren zeichnen sich durch ein hohe pH- und Temperatur-Stabilität aus. Sie gelangen meistens über die Atemwege in den Organismus. Die Vermehrung der Adenoviren ist jedoch nicht lokal begrenzt: sie können sich in den Epithelzellen des Pharynx, des Magen-DarmTrakt sowie des Auges, vor allem der Konjunktiva, nach dem lytischen Vermehrungszyklus vermehren.

Eine Infektion mit Adenoviren im Auge ruft insbesondere Keratokonjunktivitiden, Konjunktividen und schwerwiegende Keratiden, z.B. die Keratokonjunktivitis epidemica, als Virus-basierte Erkrankungen des Auges hervor. Adenoviren sind die häufigste Ursache für die Virus-basierte Konjuntivitis. Symptome sind tränende, schmerzende Augen, Lichtempfindlichkeit und Hornhauterosionen, die bis zu sechs Wochen andauern können. Weiterhin können Adenoviren das Pharyngokonjunktivalfieber auslösen.

Die Art und Schwere der Infektion hängt dabei vom auslösenden Adenovirustyp ab. Beispielsweise führen Infektionen mit den Adenoviren 5, 8 und 19 zu den schwersten Keratokonjunktividen des Auges. Infektionen mit Adenoviren sind stark kontagiös.

In den letzten Jahrzehnten sind antivirale Wirkstoffe zur Behandlung von zahlreichen Virusinfektionen, sogenannte Virostatika, entwickelt worden, die in den Vermehrungszyklus des Virus eingreifen sollen und bereits die virale Infektion der Wirtszelle oder die Vermehrung des Virus in der Wirtszelle inhibieren oder hemmen sollen.

Eine Behandlung der Virus-basierten Erkrankungen des Auges mit antiviralen Wirkstoffen ist allerdings gegenwärtig ausschließlich bei einer Infektion durch Herpesviridae, wie HSV-1, Varizella zoster Virus und Cytomegalieviren, und Influenzaviren (Typ A und Typ B) möglich. Auf die gegenwärtigen Behandlungen wird nachfolgend näher eingegangen:

### Antivirale Behandlung von Herpesviurs-basierten Erkrankungen des Auges

Herpesinfektionen des Auges werden mit oral oder topisch verabreichten Virostatika, wie Aciclovir (Zovirax®), Valacilovir (Valtrex®), (Goldblum D et al. Comparison of oral antiviral therapy with valacyclovir or acyclovir after penetrating keratoplasty for herpetic keratitis, British Journal of Ophthalmology 2008; Bd. 92:1201-1205) Gangciclovir, Trifluridin (Michael HG, Therapy of viral infections, Bull. N.Y. Acad. Med. 2008; Bd. 59, No.8: 711-720), Idoxuridin (Virungent®) (Michael HG, Therapy of viral infections, Bull. N.Y. Acad. Med. 2008; Vol. 59, No.8: 711-720), Famciclovir (Famvir®) und Cidofovir (Vistide®) behandelt. Dabei handelt es sich um Nucleosidanaloga, die in das Ergbut des Virus integriert werden und somit die RNA-Polymerase, bzw. DNA-Polymerase des Wirtes während der Virus-Replikation hemmen. Es kommt zum Kettenabbruch und somit zum Stopp der Replikation und der Virus-Vermehrung. Welches Virustatikum verabreicht wird, hängt dabei u.a. von der Art des Herpesvirus und der Tiefe der betroffenen Hornhautschichten im Auge ab. Oberflächliche Infekte durch HSV-1 werden zum Beispiel vorwiegend lokal mit Trifluridin behandelt, während die Behandlung von tieferen Schichten häufig durch die Applikation einer Aciclovir-Augensalbe erfolgt. Tieferen Schichten der Hornhaut, die durch Herpesviren infiziert sind, können jedoch grundsätzlich nur schlecht durch die genannten Virustatika erreicht werden. Bei Befall dieser Schichten ist daher meistens eine Kombination der Virustatika mit symptom-reduzierenden Pharmazeutika, wie z.B. antientzündlichen Corticosteroiden, nötig.

### Antivirale Behandlung von Influenzaviurs-basierten Erkrankungen des Auges

Influenza Viren A und Influenza B Viren werden durch Neuramidasehemmer, wie Zanamivir (Relenza®) und Oseltamivir (Tamiflu®), gehemmt. Das Enzym Neuramidase hilft den neu gebildeten Viruszellen, sich von der Wirtszelle abzuknospen, damit sie weitere Zellen infizieren können. Durch die Hemmung der Neuramidase wird die Virusvermehrung unterdrückt und somit die weitere Infektion gestoppt. Neuramidasehemmer können auch prophylaktisch eingesetzt werden, um einer Virusinfektion mit Influenza A und Influenza B, insbesondere im Falle von Epidemien, vorzubeugen (Sugrue RI et al., Antiviral Drugs fort he Control of Pandemic Influenza Virus. Annals Academy of Medicine, 2008, Bd 37, 518-524.).

Für Influenza Virus A sind darüber hinaus M2-Kanalblocker bekannt (Amantadin, Rimantadin). Diese verhindern das "Uncoating", d.h. die Freisetzung der Virus-Nucleocapside in das Cytoplasma in der Wirtszelle, und somit die Infektion der Wirtszelle, wodurch und die Virusvermehrung abgebrochen wird (Sugrue RI et al.. Antiviral Drugs fort he Control of Pandemic Influenza Virus. Annals Academy of Medicine, 2008, Bd. 37, 518-524).

Sowohl Neuramidase-Hemmer als auch M2-Kanalblocker werden jedoch aufgrund ihrer hohen Nebenwirkungen nur zur Therapie von Virus-basierten Augenerkrankungen eingesetzt, wenn eine schwerwiegende Influenzainfketionen diagnostiziert wird.

Die aufgeführten antiviralen Therapiemöglichkeiten der Virus-basierten Erkrankungen der Augen haben erhebliche Nachteile:
- Bei einer systemischen Verabreichung ist die für eine eine effektive Behandlung erforderliche Dosis relativ hoch verbunden mit starken Nebenwirkungen für den behandelten Organismus, wie beispielsweise unspezifische Immunantworten und Autoimmunreaktionen. Für Fall von Aciclovir, Amantadin und Rimantadin z.B. sind zahlreiche Nebenwirkungen aus der Literatur bekannt. Weder eine Langzeit-Therapie noch Wiederholungs-Therapien sind daher ratsam und einem Patienten zuzumuten;
- Bei der topischen Applikation ist die Menge an Wirkstoff (Virostatikum), welche pro Zeiteinheit in dem Gebiet der Virusinfektion freigesetzt und bioverfügbar werden kann, sehr gering. Diese geringe Bioverfügbarkeit des Virostatikums stellt ein wesentliches Hindernis für eine effektive topische Therapie dar. Für den Fall des nur schwer wasserlöslichen Aciclovir, z.B., liegt die geringe Bioverfügbarkeit beispielsweise an dem schlechten perkutanen Transport des Wirkstoffs. Auch verschiedene chemische Modifikationen von Virostatika im Rahmen von Pro-Drug Konzepten für eine verbesserte Wirkstoffzufuhr von Virostatika haben zu keiner Verbesserung dieses Phänomens geführt.
- Die beschriebenen Zytostatika sind sehr spezifisch und können somit nicht gegen verschiedene Viren zur Behandlung Virus-basierter Erkrankungen des Auges eingesetzt werden. Bei vielen Virus-basierten Erkrankungen des Auges, z.B. der viralen Konjunktivitis, kann anhand der Symptomatik nicht auf den Virus-Typus, z.B. eine Influenza Infektion, geschlossen werden. In diesen Fällen wäre eine Therapie mit einem Virostatikum, das gegen nur einen Virus-Typus wirkt, zu einer großen Wahrscheinlichkeit erfolglos und würde darüber hinaus beschriebene Nebenwirkungen hervorrufen.

Weitere ernste Nachteile sind, dass die beschriebenen antiviralen Therapiemöglichkeiten der Virus-basierten Erkrankungen des Auges zu weiteren erheblichen Nebenwirkungen führen können und, wie beispielsweise im Fall von M2-Kanalblockern, zu resistenten Virusstämmen führen können, die eine Behandlung derartiger Erkrankungen mit solchen Wirkstoffen und eventuell sogar weiteren Wirkstoffen für zukünftige ausschließt.

Mit den beschriebenen antiviralen Therapien ausschließlich Infektionen der Augen behandelt werden, die durch die beschriebenen Herpesviren und Influenzaviren ausgelöst wurden. Eine antivirale Behandlung von Erkrankungen des Auges, die durch andere Viren, wie z.B. Adenoviren, Mumpsviren, Masernviren, Rötelviren, Epstein-Barr-Virus, Parainfluenzaviren, Picornaviren (Rhinoviren, Coxackie-Virus A, Coxsackie-Virus B, Echo-Viren), Molluscum contagiosum Virus (Pox-Virus), Vacciniaviren, Polioviren, Tollwutviren, humane Immundefizienz Viren und Rifttal-Fieber-Virus verursacht werden, gibt es nicht.

Alternativen zu den gegenwärtig bekannten antiviralen Wirkstoffen, die die im Stand der Technik bekannten Nachteile überwinden könnten, sind ausschließlich symptomatische Behandlungen, z.B. von entzündlichen Reaktionen auf die Virusinfektion mit topischen Vasokontriktoren (z.B. Naphazolin) oder Steroiden (z.B. Vexol, Flarex), jedoch keine therapeutische Behandlung der Erkrankung selbst).

Alternativen zu bekannten antiviralen Wirkstoffen zur Prophylaxe und/oder Therapie von Virus-basierten Erkrankungen des Auges, die die im Stand der Technik bekannten Nachteile überwinden, sind ausschließlich prophylaktische Impfungen gegen die entsprechenden Viren. Effektive Impfungen gegen Viren, die das Auge infizieren können, sind nach dem derzeitigen Stand der Technik allerdings nur für die Influenza Viren (Typ A und B) sowie gegen Pocken-, Röteln, Masern- und Mumpsvirus bekannt.

Die meisten Viren, wie z.B. die Influenzaviren, sind darüber hinaus ständigen Mutationen unterworfen, so dass Impfstoffe stets neu entwickelt werden müssen. Darüber hinaus sind die Impfungen nicht bei allen geimpften Patienten wirksam. Therapien viral bedingter Erkrankungen können demnach keinesfalls durch Impfungen ersetzt werden.

Weitere Alternativen zu den im Stand der Technik offenbarten antiviralen Wirkstoffen bzw. Virostatika, zur Prophylaxe und/oder Therapie von Virus-basierten Erkrankungen des Auges, welche die im Stand der Technik bekannten Nachteile überwinden, gibt es nicht.

Es besteht daher Bedarf, verbesserte und verträglichere antivirale Wirkstoffe zur Prophylaxe und/oder Therapie von Virus-basierten Erkrankungen des Auges zu entwickeln, die in den Replikations- und/oder Vermehrungszyklus des Virus einzugreifen, und die vorzugsweise bereits die virale Infektion der Wirtszelle hemmen oder inhibieren.

Darüber hinaus besteht Bedarf, antivirale Wirkstoffe zu identifizieren, welche die Infektion, Replikation und/oder Vermehrung verschiedener Virusstämme oder Viursspezien gleichzeitig inhibieren. Dies ist besonders wichtig, da bei den meisten Erkrankungen des Auges symptomatisch nicht zwischen den verschiedenen Virusstämmen oder Virusspezien, welche die Infektion ausgelöst haben, unterschieden werden kann.

Aufgabe der Erfindung ist es demnach, verbesserte antivirale Wirkstoffe zur Prophylaxe und/oder Therapie von Virus-basierten Erkrankungen des Auges bereitzustellen.

Diese Aufgabe wird durch die vorliegende Erfindung gelöst. Die Erfindung betrifft in ihrem ersten Gegenstand Deuteriumoxid zur Verwendung für die Prophylaxe und/oder Therapie von Virus-basierten Erkrankungen des Auges, wobei es sich bei der Virus-basierten Erkrankung des Auges um Keratokonjunctivitis epidemica, Keratokonjunktivitis sicca, hemorrharische Konjunktivitis, follikuläre Konjunktivitis, Pharyngokonjunktivalfieber, Blepharitis, Blepharokonjunktivitis, Keratitis dendritica, Keratitis interstitialis, Edotheliitis, Keratitis disciformis, Ophthalmia neonatorum, Dakryoadentitis, akute Dakryoadentitis, Dakryocystose, Episkleritis, Skleritis, Chorioditis, Chorioretinits, Retinitis, akute nekrotisierende Retinitis, Uveitis anterior, Uveitis intermediate und/oder Uveitis posterior handelt, und wobei die Replikation der Virusnukleinsäure und damit die Virusvermehrung gehemmt oder inhibiert wird

Eine bevorzugte Ausführungsform der Erfindung betrifft die Verwendung von Deuteriumoxid zur Prophylaxe und/oder Therapie von Virus-basierten Erkrankungen des Auges, wobei es sich bei der Virus-basierten Erkrankungen des Auges um Konjunktividen, Keratokonjunktividen und/oder Uveitiden handelt.

Eine weitere bevorzugte Ausführungsform der Erfindung betrifft Deuteriumoxid nach Anspruch 1, wobei Deuteriumoxid in einer Konzentration von mehr als 20% bezogen auf den Gesamtwasserhaushalt der Zelle verabreicht wird.

Verschiedene Erkrankungen des Auges können auch parallel oder nacheinander auftreten. Eine offenbarte Ausführungsform der vorliegenden Erfindung betrifft daher die Verwendung von Deuteriumoxid zur Prophylaxe und/oder Therapie von Virus-basierten Erkrankungen des Auges, wobei es sich um eine Kombination von zwei oder mehreren Virus-basierten Erkrankungen des Auges handelt. Bevorzugt treten diese zwei oder mehreren Virus-basierten Erkrankungen des Auges gleichzeitig oder nacheinander, insbesondere als Folge der ersten oder einer der vorangehenden Erkrankung(en), auf. Bei einer solchen Kombination von zwei oder mehreren Virus-basierten Erkrankungen des Auges handelt es sich in einer offenbarten Ausführungsform um Rhinokonunktivitis.

Unter "Virus-basierten Erkrankungen des Auges" im Sinne der Erfindung sind Erkrankungen des Auges zu verstehen, die von einem Virus durch eine Virusinfektion, hervorgerufen werden. Die vorliegende Erfindung offenbart nicht nur die Therapie, sondern auch die Prophylaxe von Virus-basierten Erkrankungen des Auges, wie oben bereits definiert. Unter Virus-basierten Erkrankungen des Auges sind daher erfindungsgemäß ebenfalls solche Erkrankungen des Auges zu verstehen, die einer prophylaktischen Behandlung von Virus-basierten Erkrankungen des Auges im Sinne der Erfindung zuzuordnen sind. Hierzu gehören beispielsweise auch einer Virus-basierten Erkrankung des Auges typischerweise vorangehenden Erkrankungen. Hierzu gehört insbesondere Keratokonjunktivitis sicca sowie hiervon abgeleitete Indikationen und/oder Unterformen, wie z.B. Keratis superficialis und Keratis filiformis, die erfindungsgemäß demnach ausdrücklich als Virus-basierte Erkrankung des Auges bezeichnet werden. Eine bevorzugte erfindungsgemäße Verwendung von D20 betrifft somit die Verabreichung von D20 bei auftretender Keratokonjunktivitis sicca. Die Offenbarung in der vorliegenden Erfindung unter dem allgemein verwendeten Begriff "Keratokonjunktivitis" schließt alle Formen der Keratokonjunktivitis als Virus-basierte Erkrankungen des Auges ein.

Unter den Begriff "Auge" im Sinne der Erfindung fallen alle Gewebe-, insbesondere Haut- und Zelltypen, die von einem Virus befallen werden können. Diese werden erfindungsgemäß auch als "Organe des Auges" oder "Organe" bzw. "zu behandelnde Organe des Auges" oder "zu behandelnde Organe" bezeichnet. Hierunter fallen sowohl oberflächliche als auch tiefere Hautschichten, vor allem die Lidhaut, Bindehaut, die oberen Schichten der Hornhaut, sowie tiefere Schichten der Hornhaut, die Aderhaut, die Lederhaut und weitere Bereiche der Uvea, die Häute und Schleimhäute des Auges, wie der Tränensäcke, Tränenkanälchen und Tränendrüsen. Insbesondere werden die Epithelzellen dieser vorstehend beschriebenen Häute durch das Virus infiziert, die ebenfalls unter den Begriff "Auge" fallen.

Zahlreiche der vorgenannten Organe oder Organe des Auges stellen von außen zugängliche Bereiche des Auges dar. "Von außen zugänglich" im Sinne der vorliegenden Erfindung bedeutet, dass die Organe, insbesondere deren Schleimhäute, durch äußere Applikation oder Verabreichung des erfindungsgemäßen Wirkstoffs Deuteriumoxid (D20), d.h. nicht systemisch, erreichbar sind.

So eine "äußere" Verabreichung kann erfindungsgemäß beispielsweise über eine D20-haltige Augensalbe (D20-Augensalbe), D20-Augengel, D2O-Augentropfen oder durch Spülen der entsprechenden Organe oder Bereichen der Organe mit D20 oder durch Verabreichung von flüssigem D20 oder D20-Gelen oder D20-Hydrogelen erfolgen. Eine umfassende und detaillierte Beschreibung der erfindungsgemäßen Verabreichungswege von D20 wird untenstehend aufgeführt. Eine "innere" Verabreichung kann erfindungsgemäß durch Injektion von D20 in das Auge erfolgen und wird vorgenommen, um an hintere Bereiche es Auges zu kommen.

Organe oder Bereiche solcher Organe die erfindungsgemäß als "von außen zugänglich" zu definieren sind, sind z. B. Lidhaut, Bindehaut, die oberen Schichten der Hornhaut, die Häute und Schleimhäute der Tränensäcke, Tränenkanälchen und Tränendrüsen.

Unter "Virusinfektion" im Sinne der Erfindung ist das aktive oder passive Eindringen eines Virus in einen Organismus, wie Pflanze, Tier oder Mensch, und deren Vermehrung in diesem Organismus zu verstehen. Ein solcher Organismus wird erfindungsgemäß als "Wirt" bezeichnet und umfaßt Wirbeltiere, insbesondere Säugetiere, vor allem Mensch, Pferd, Schwein, Rind, Ziege, Schaf, Katze und Hund. Die Zellen eines solchen Wirtes werden als "Wirtszellen" bezeichnet. Ein "zu behandelnder Organismus" im Sinne der vorliegenden Erfindung ist ein solcher Wirt, der an einer Virus-basierten Erkrankung des Auges entweder leidet und therapeutisch behandelt wird oder im Hinblick auf eine solche Erkrankung prophylaktisch behandelt wird, wobei der Mensch hierbei ein besonders bevorzugter Wirt ist. Ein "zu behandelndes Organ" im Sinne der Erfindung ist ein Organ eines solchen vorstehend definierten zu behandelnden Organismus.

Im Sinne der Erfindung, werden die Begriffe "Virusinfektion", "Infektion durch Viren", "virale Infektion" und "Infektion" synonym verwendet und bezeichnen alle eine durch einen Virus erfolgte Infektion.

Es sind zahlreiche Viren bekannt, die eine erfindungsgemäße Virus-basierte Erkrankung des Auges hervorrufen. Einige dieser Viren sowie ihr Infektionsweg und pathologisches Erscheinungsbild, also der durch sie hervorgerufene Erkrankung, wurde in der vorliegenden Beschreibung bereits näher beschrieben. Es betrifft daher eine bevorzugte Ausführungsform der Erfindung die Verwendung von Deuteriumoxid zur Prophylaxe und/oder Therapie von Virus-basierten Erkrankungen des Auges, wobei es sich bei dem Virus um ein Virus der Familie Poxviridae, Paramyxoviridae, Coronaviridae, Chordopoxviridae, Togaviridae, Adenoviridae und/oder Picornaviridae handelt.

Besonders bevorzugt handelt es sich bei dem Virus um ein Virus der Gattung Cytomegalievirus, Chordopoxvirus, Rubivirus, Respirovirus, Rubulavirus, Adenovirus, Mastadenovirus, Orthopoxvirus, Enterovirus und/oder Rhinovirus.

Insbesondere bevorzugt handelt es sich bei dem Virus um ein Virus der Art humanes Cytomgalievirus (HMZV), Epstein-Barr-Virus, Vacciniavirus, Rubivirus, Rötelvirus, Rubellavirus, Parainfluenzavirus 1, Parainfluenzavirus 2, Parainfluenzavirus 3, Parainfluenzavirus 4, Masernvirus, Mumpsvirus, humanes Adenovirus A, humanes Adenovirus B, humanes Adenovirus C, humanes Adenovirus D, humanes Adenovirus E, humanes Adenovirus F, Echovirus, Cosackievirus A, Cosackievirus B, Rhinovirus, Tollwutvirus, Respiratorisches Synzytialvirus und/oder Rifttal-Fieber-Virus.

Erfindungsgemäß werden die Begriffe "Virustyp" und "Virusart" synonym verwendet.

Die Begriffe "Prophylaxe und/oder Therapie" bezeichnen erfindungsgemäß jede für die Behandlung einer Virus-basierten Erkrankung des Auges Haut geeignete Maßnahme, die entweder eine vorbeugende Behandlung einer solchen sich abzeichnenden Erkrankung bzw. deren sich abzeichnende Symptome darstellt (Prophylaxe) oder die Behandlung der ausgebrochenen Erkrankung und/oder die Vermeidung eines Wiederausbrechens einer solchen Erkrankung, beispielsweise nach einem abgeschlossenen Behandlungszeitraum darstellt (Therapie).

Im Allgemeinen erfolgt eine wirksame Prophylaxe und/oder Therapie einer Virus-basierten Erkrankung des Auges insbesondere durch topische oder systemische Verabreichung eines pharmazeutischen Wirkstoffes bzw. eines antiviralen Wirkstoffes.

Unter "topisch" bzw. "topischer Applikation" oder "topischer Verabreichung" im Sinne der Erfindung ist zu verstehen, dass ein pharmazeutischer bzw. antiviraler Wirkstoff, erfindungsgemäß D20, - im Gegensatz zur systemischen Aufnahme - nicht in den Blutkreislauf des zu behandelnden Organismus aufgenommen wird, sondern nur lokal, insbesondere oberflächlich, auf/in ein zu behandelndes Organ des Auges, insbesondere auf/in dessen Epithelzellen oder Schleimhaut, aufgetragen, aufgebracht oder eingebracht wird, vorzugsweise als Formulierung in Form von Tropfen, d.h. in flüssiger Form, wie beispielsweise als Augenspülung oder Augentropfen, oder einem Hydrogel, Gel, einer Salbe oder Creme.

Unter "systemischer" Verabreichung im Sinne der Erfindung ist zu verstehen, dass ein pharmazeutischer bzw. antiviraler Wirkstoff in den Blutkreislauf des zu behandelnden Organismus aufgenommen wird. Die topische Verabreichung eines pharmazeutischen bzw. antiviralen Wirkstoffs ist grundsätzlich nebenwirkungsärmer als die systemische Verabreichung, da bei der topischen Verabreichung nur lokal, d.h. in einem definierten und begrenzten Bereich des zu behandelnden Organs eines Organismus, hohe Wirkstoffkonzentrationen erreicht werden und nicht der gesamte Organismus, genauer dessen Blutkreislauf, dem Wirkstoff ausgesetzt ist, wie es bei der systemischen Verabreichung der Fall ist.

Ein solcher zur Prophylaxe und/oder Therapie einer Virus-basierten Erkrankung des Auges topisch verabreichter pharmazeutischer Wirkstoff bzw. antiviraler Wirkstoff sollte mindestens eine, vorzugsweise mehrere, der nachstehenden Eigenschaften aufweisen:
a) lokal spezifische Anwendbarkeit durch topische Verabreichung über einen beliebig langen Zeitraum und mit hoher permucosidaler Transportkinetik des Wirkstoffs. Diese wird erreicht durch den gezielten Transport des Wirkstoffes in definierte Bereiche des zu behandelnden Organs, insbesondere in die Zellen, vor allem Epithel- und Schleimzellen, durch die Wahl einer geeigneten Formulierung des Wirkstoffs (beispielsweise als Spülung),
b) Retardierung des transepithelialen Transports des Wirkstoffs in den Blutkreislauf, d.h. in die Blutgefäße,
c) weitgehend homogene Wirkstoffverteilung im Bereich des Wirkortes (zu behandelndes Organ) sowie die Vermeidung von lokalen Überkonzentrationen;
d) virostatische Wirkung, d.h. eine die Vermehrung von Viren hemmende oder inhibierende Wirkung, auf die Zellen im behandelten Bereich des Organs. (d.h. die Virusvermehrung durch den wirtszelleigenen Replikationsapparat wird gehemmt oder inhibiert).
e) weitgehende Toleranz des antiviralen Wirkstoffs durch die gesunden (vom Virus nicht infizierten) Zellen im Hautgewebe sowie den Blutkreislauf zur Vermeidung von Nebenwirkungen, insbesondere auch im Hinblick auf Immunreaktionen.

Erfindungsgemäß wurde festgestellt, dass Deuteriumoxid (D20) alle der vorgenannten Eigenschaften erfüllt. Darüber hinaus weist D20 gegenüber den im Stand der Technik bekannten antiviralen Wirkstoffen, insbesondere den Virostatika, deutliche Vorteile auf, beispielsweise weitaus geringere bzw. keine Nebenwirkungen, erhöhte Bioverfügbarkeit und universale Anwendbarkeit auf alle Viren, die Infektionen des Auges verursachen.

Erfindungsgemäß wurde weiterhin festgestellt, dass Deuteriumoxid ein effektiver antiviraler Wirkstoff mit potentieller Langzeitwirkung ist und sowohl für Kurz-, Langzeit- und Wiederholungs-Therapien von Virus-basierten Erkrankungen des Auges geeignet ist. Erfindungsgemäß ist Deuteriumoxid, nachfolgend auch als D20 bezeichnet, ein pharmazeutischer bzw. antiviraler Wirkstoff.

Unter dem Begriff "pharmazeutischer Wirkstoff" wird erfindungsgemäß jede(s) beliebige anorganische oder organische Molekül, Substanz oder Verbindung verstanden, das (die) eine pharmakologische Wirkung aufweist. Der Begriff "pharmazeutischer Wirkstoff" wird hierin mit dem Begriff "Arzneimittel" synonym verwendet. Zu den pharmazeutischen Wirkstoffen im Sinne der Erfindung gehören auch antivirale Wirkstoffe, einschließlich D20.

Der Begriff "antiviraler Wirkstoff" im Sinne der Erfindung definiert einen Wirkstoff, der zur Behandlung von Virus-basierten Erkrankungen, und insbesondere Virus-basierten Erkrankungen des Auges, verabreicht wird. Vorzugsweise ist ein solcher antiviraler Wirkstoff geeignet, bereits die virale Infektion der Wirtszelle und/oder die Vermehrung der viralen Nukleinsäure in der Wirtszelle und/oder die Vermehrung des Virus zu inhibieren oder zu hemmen. Sofern ein antiviraler Wirkstoff die Virusvermehrung hemmt oder inhibiert, wird auch der Begriff "virostatischer Wirkstoff" verwendet. Die Begriffe "pharmazeutischer Wirkstoff" und "antiviraler Wirkstoff" werden im Sinne der Erfindung synonym verwendet.

"Zellen" im Sinne der Erfindung sind Wirbeltierzellen, insbesondere Säugetierzellen und vor allem humane Zellen (Zellen des Menschen), und zwar Hautzellen, vor allem Epithelzellen, Schleimzellen und Schleimhautzellen. Bei "Wirtszellen" handelt es sich erfindungsgemäß um Zellen, die von einem Virus infiziert wurden oder infiziert werden können. Bei den definierten Zellen und Wirtszellen der Erfindung handelt es sich um solche des Auges.

Die Eigenschaften von D20 sowie dessen erfindungsgemäße Verwendung als antiviraler Wirkstoff zur Prophylaxe und/oder Therapie von Virus-basierten Erkrankungen des Auges werden nachfolgend erläutert:

Deuteriumoxid (D20), häufig auch als "schweres Wasser" bezeichnet, ist eine dem "natürlichen Wasser" H2O in seinen physikalischen Eigenschaften äußerst ähnliche Substanz. Deuteriumoxid (D20) und Wasser (H2O) unterscheiden sich physikalisch durch die Substitution der Wasserstoffatome von H2O durch Deuteriumatome, wobei D20 eine ca. 10% höhere Dichte und eine ca. 25% höhere Viskosität aufweist. Darüber hinaus sind Schmelz- und Siedetemperaturen von D20 höher als für H2O. Ein detaillierter Vergleich der Eigenschaften wird im Handbook of Chemistry and Physics, Sektion 6, gegeben (Handbook of Chemsitry and Physics, David R. Lide, Editor, 79. Auflage, 1998 CRC Press, Boca Raton, USA) dargestellt.

Neben den physikalisch ähnlichen Eigenschaften von H2O und D20 bestehen bedeutsame physiologische Unterschiede (siehe u.a. Kushner DJ et al., Pharmacological uses and perspectives of heavy water and denatured compounds., Can J Physiol Pharmacol. 1999 Feb; 77(2): 79-88.). D20 weist ab einer bestimmten Konzentration in einer Zelle, bei tierischen Zellen von mehr als 20 - 25 %, eine Wirkung auf enzymatische Reaktionen auf. Enzymatisch gesteuerte Reaktionen werden zunehmend verändert, insbesondere gehemmt oder inhibiert. Eine Ursache hierfür wird in der höheren Bindungsstärke der Wasserstoffbrückenbindungen, wenn das Wasserstoffatom der Bindung durch ein Deuteriumatom substituiert ist, gesehen. Sowohl in wässrigen Lösungen von H2O und D20, als auch bei der Bindung von Wasser an organische Moleküle, tritt diese erhöhte Bindungsstärke auf (Cuma M, Scheiner S, Influence of Isotopic Substitution on Strength of Hydrogen Bonds of Common Organic Groups, Journal of Physical Organic Chemistry, 1997 Band 10, 383-395). Diese erhöhte Bindungsstärke von Wasserstoff-Brückenbindungen (H-Brücken) in dem Fall, dass das Wasserstoffatom der Bindung durch ein Deuteriumatom substituiert wird sowie die verringerte Austauschgeschwindigkeit von D20 gegenüber H2O (König, S.,et al. Molecular dynamics of water in oriented multilayers studied by quasi-elastic neutron scattering and deuterium-NMR relaxation. 1994. J.Chem. Phys. 100, 3307-3316) hat für die Bindung von D20 an für H-Brücken geeignete Bindungsstellen zwei unmittelbare Konsequenzen: a) Die Aufenthaltswahrscheinlichkeit von D20 an Oberflächen mit Bindungsmöglichkeiten für H-Brücken wird gegenüber der von H2O deutlich erhöht. b) Die Hydratation der unter a) genannten Oberflächen erhöht sich und die Ablösung von D20 (z.B. durch Verdunstung) ist energetisch erschwert, was wiederum die Nachhaltigkeit der Hydratation erhöht.

Unter "Hydratation" (auch Hydration) ist erfindungsgemäß die Anlagerung von D20-Molekülen anstelle von oder zusätzlich zu H2O-Molekülen an eine gegebene Oberfläche, und zwar die zu behandelnden Organe des Auges, zu verstehen. Eine Hydratation im Sinne der Erfindung kann auch als D2O-Hydratation bezeichnet werden.

Unter "Hydratationsgrad" oder "Hydratationsmaß" ist erfindungsgemäß die Anzahl der an eine gegebene Oberfläche, und zwar die zu behandelnden Organe des Auges, maximal über H-Brücken im Zeitmittel bindenden D20-Molekülen anstelle von oder zusätzlich zu H2O-Molekülen zu verstehen. Hydratationsgrad oder Hydratationsmaß im Sinne der Erfindung kann auch als D2O-Hydratationsgrad oder D2O-Hydratationsmaß bezeichnet werden.

Unter "Nachhaltigkeit der Hydratation" soll hierbei die Aktivierungsenergie zur Ablösung eines durch H-Brücken gebundenen D20- bzw H2O-Moleküls von einer Oberfläche, und zwar der zu behandelnden Organe des Auges, verstanden werden, wobei eine höhere Aktivierungsenergie eine höhere Nachhaltigkeit bedeutet. Die Nachhaltigkeit der Hydratation im Sinne der Erfindung kann auch als Nachhaltigkeit der D2O-Hydratation bezeichnet werden.

Die Schleimhäute als erfindungsgemäß definierte (zu behandelnde) Organe des Auges sind Beispiele für besonders stark hydratisierte Membranoberflächen, bei denen die Zahl der H-Brücken Bindungsmöglichkeiten durch die Anwesenheit von Glykolipiden und Glykoproteinen gegenüber anderen Membranen (z.B. der Hautoberfläche) massiv erhöht ist. Eine leichte Erhöhung von Hydratation und/oder der Nachhaltigkeit der Hydratation hat in diesem Fall besonders signifikante Folgen, die sich vorteilhaft auf die erfindungsgemäße Verwendung von D20 auswirkt.

Es ist allgemeines Fachwissen, dass eine Zelle abhängig von dem Grad ihrer jeweiligen metabolischen Aktivität unterschiedliche Mengen H2O aufnimmt. Eine Zelle, die von einem Virus infiziert wird, eine sog. Wirtszelle, und in der eine Vermehrung des Virus stattfindet, weist eine weitaus höhere metabolische Aktivität auf als eine nicht infizierte Zelle desselben Zelltyps des umliegenden Gebiets. Der Grund hierfür ist, dass die Wirtszelle nicht nur ihre eigene Replikation, sondern auch die Replikation des Virus bewirkt. Da eine erhöhte metabolische Aktivität von Zellen mit einer erhöhten Wasseraufnahme korreliert, nehmen Virus-infizierte Wirtszellen deutlich mehr Wasser (H2O) auf als nicht infizierte Zellen. Aufgrund der ähnlichen physikalischen Eigenschaften von H2O und D20, wird D20 von Zellen parallel zu H2O (wenn D20 und H2O verfügbar sind) oder anstelle von H2O (wenn nur D20 verfügbar ist) aufgenommen.

### Wirkung von D2O auf die Replikation und Vermehrung eines Virus

Wie vorstehend ausgeführt ist bekannt, dass enzymatische Reaktionen in einer Zelle durch D20 in ausreichender Konzentration verändert werden können. Wie vorstehend erläutert, nehmen Zellen, und Virus-infizierte Zellen in erhöhtem Ausmaß, D20 parallel oder anstelle von H2O auf. Wird demnach an eine Virus-infizierte Zelle D20 in einer erfindungsgemäß "ausreichenden Konzentration", d.h. mehr als 20% bezogen auf den Gesamtwassergehalt einer Zelle, verabreicht, hat dies eine Hemmung bzw. Inhibierung enzymatischer Reaktionen in der Wirtszelle zur Folge. Hierzu gehört insbesondere die Hemmung oder Inhibierung der DNA-Polymerase (Takeda H et al., Mechanisms of cytotoxic effects of heavy water (deuterium oxide: D2O) on cancer cells, Anticancer Drugs. 1998 Sep;9(8):715-25). Als Folge hiervon wird die DNA-Replikation in einer Zelle gehemmt bzw. inhibiert. Nicht infizierte, gesunde Zellen in dem die Virus-infizierten Zellen umgebenden Bereich (Gebiet), nehmen D20 bzw. H2O aufgrund ihrer - im Vergleich zur infizierten Zelle - geringeren metabolischen Aktivität in normalem Maß auf, so dass in diesen keine oder eine nur sehr geringe, vernachlässigbare Hemmung oder Inhibierung enzymatischer Reaktionen erfolgt.

Erfindungsgemäß ist nunmehr festgestellt worden, dass, wenn eine Zelle mit einem Virus infiziert wird (Wirtszelle), durch die Hemmung bzw. Inhibierung der wirtszelleigenen DNA-Polymerase auch die Virus-DNA nicht repliziert wird, da deren Replikation ebenfalls durch die Beteiligung der Wirtszell-DNA-Polymerase erfolgt. Im Fall von RNA-Viren erfolgt die enzymatische Hemmung bzw. Inhibierung durch D20 auch für Synthese der reversen Transkriptase, die zunächst, durch Virusgene kodiert, in der Wirtszelle synthetisiert werden muss, um die Virus-RNA in Virus-DNA umzuschreiben, die dann wiederum von der DNA-Polymerase der Wirtszelle repliziert wird. Eine Hemmung bzw. Inhibierung bestimmter enzymatischer Reaktionen der Wirtszelle durch D20 hemmt bzw. inhibiert somit auch die Replikation und somit auch die nachfolgende Vermehrung (wie oben definiert) eines Virus nach einer Virusinfektion.

### Wirkung von D2O auf die Zellteilung Virus-infizierter Zellen

Ein weiterer wichtiger Aspekt der Erfindung, der auf der beschriebenen erhöhten Bindungseigenschaft von D20 basiert, ist, dass bei Verabreichung von D20 in ausreichender Konzentration, d.h. mehr als 20%, bezogen auf den Gesamtwassergehalt einer Zelle, die Zellteilung gehemmt oder inhibiert wird. Dies geschieht höchstwahrscheinlich, zusätzlich zu der erläuterten Hemmung bzw. Inhibierung der DNA-Replikation, durch die Hemmung bzw. Inhibierung der Mitose im Zyklus der Teilung tierischer Zellen (Laissue JA et al. Survival of tumor-bearing mice exposed to heavy water or heavy water plus methotrexate, Cancer Research, 1982, Bd. 42, (3) 1125-1129). Dies ist erfindungsgemäß von entscheidender Bedeutung für den Latenzzustand bei Virus-basierten Erkrankungen des Auges, bei dem die Viren im Ruhezustand (Latenz) während des oben näher beschriebenen lysogenen Zyklus voliegen. In diesem Zustand wird das Virusgenom in das Wirtszellgenom integriert und bei Teilung der Wirtszelle zusammen mit dem Wirtsgenom auf die Wirtszellnachkommen übertragen. Bei einer Hemmung bzw. Inhibierung der Zellteilung wird so eine Übertragung des Virus auf neue Zellen verhindert.

Erfindungsgemäß erfolgt demnach eine Hemmung oder Inhibierung der Virusvermehrung durch die Verwendung von D20 zur Prophylaxe und/oder Therapie von Virus-basierten Erkrankungen des Auges. Diese Hemmung oder Inhibierung der Virusvermehrung durch D20 als antiviraler Wirkstoff erfolgt erfindungsgemäß insbesondere durch
- die Hemmung oder Inhibierung der Replikation der Virus-Nukleinsäure und somit der Vermehrung des Virus und/oder
- die Hemmung oder Inhibierung der Wirtszellteilung und somit der Vermehrung des Virus im lysogenen Zyklus.

Unter dem Begriff "hemmen" bzw. "Hemmung" gemäß der Erfindung ist zu verstehen, dass die Replikation von Virus-Nukleinsäuren (Virus-DNA oder Virus-RNA), die Virusvermehrung und/oder die Zellteilungsrate von Wirtszellen der Erfindung verlangsamt und/oder herabgesetzt wird, bevorzugt bis zu 6%, vorzugsweise bis zu 10%, vorzugsweise bis zu 15%, ebenfalls bevorzugt bis zu 20%, stärker bevorzugt bis zu 25%, stärker bevorzugt bis zu 30%, ebenfalls stärker bevorzugt bis zu 35%, ebenfalls stärker bevorzugt bis zu 40%, ebenfalls stärker bevorzugt bis zu 45%, ebenfalls stärker bevorzugt bis zu 50%, noch stärkster bevorzugt bis zu ca. 55%, noch stärkster bevorzugt bis zu ca. 60%, weiterhin stärker bevorzugt bis zu 65%, ebenfalls stärker bevorzugt bis zu 70%, ebenfalls stärker bevorzugt bis zu 75%, ebenfalls stärker bevorzugt bis zu 80%, ebenfalls stärker bevorzugt bis zu 85%, noch stärker bevorzugt bis zu 90%, am stärksten bevorzugt bevorzugt bis zu 94% gegenüber der Replikations- oder Vermehrungsrate des Virus bzw. der Wirtzszellteilungsrate ohne Verabreichung von D20.

Der Begriff "inhibieren" bzw. "Inhibierung" gemäß der Erfindung bedeutet, dass die Replikation von Virusnukleinsäuren (Virus-DNA oder Virus-RNA), die Virusvermehrung und/oder die Zellteilungsrate von Wirtszellen der Erfindung verhindert wird, bevorzugt bis zu 95%, noch stärker bevorzugt bis zu 98%, am stärksten bevorzugt um bis zu 100% (und damit vollständig) gegenüber der Replikations- oder Vermehrungsrate des Virus bzw. der Wirtzszellteilungsrate ohne Verabreichung von D20.

Eine solche, auf der Hemmung oder Inhibierung der Replikation von Virusnukleinsäuren (viraler Nukleinsäuren) und/oder der Virusvermehrung basierende antivirale und virostatische Wirkung von D20, ist bislang im Stand der Technik nicht beschrieben.

D20 weist darüber hinaus gegenüber bekannten aniviralen und virostatischen Wirkstoffen zur Behandlung von Virus-basierten Erkrankungen des Auges erhebliche Vorteile auf, und zwar vor allem durch folgende Eigenschaften von D20:
1) D20 ist nicht Virus-spezifisch und kann die Replikation und/oder Vermehrung aller erfindungsgemäß beschriebenen Viren hemmen oder inhibieren. Dies ist von besonderem Vorteil, da die meisten Virus-basierten Erkrankungen des Auges, wie oben beschrieben, von verschiedenen Virusarten (auch Virustypen) ausgelöst werden können und zudem keine symptomatische Unterscheidung möglich ist. D20 kann demnach als sog. "Breitband"-Virostatikum zur Prophylaxe und/oder Therapie von allen Virus-basierten Erkrankungen des Auges verabreicht werden.
2) Durch die erfindungsgemäße topische Verabreichung von D20, z.B. durch Aufbringen von D2O-Augentropfen, D20-Augensalbe, D20-Augencreme, D20-Augensalbe oder durch Spülung mit einer D20-Lösung, kann eine zur therapeutischen Wirksamkeit ausreichend hohe Konzentration (d.h. mehr als 20% bezogen auf den Gesamtwassergehalt einer Zelle) von D20 in den Epithelzellen der zu behandelnden Organe des Auges erreicht werden, ohne dass andere, nicht Virus-infizierte, Organe des Organismus ähnlich hohen Konzentrationen von D20 ausgesetzt werden, wie es bei der systemischen Verabreichung erfolgt. Damit ist ein entscheidendes, im Stand der Technik diskutiertes Problem zur Erreichung von therapeutisch wirksamen D2O-Konzentrationen am Wirkungsort (d.h., mehr als 20% D20 bezogen auf den Gesamtwassergehalt der Zelle) ohne starke Nebenwirkungen für andere, gesunde Organe oder gesundes umliegendes Gewebe gelöst.
3) Der Aggregatzustand von D20 ist erfindungsgemäß bei einer topischen Verabreichung flüssig oder liegt in einer fest Formulierung, wie einer Salbe, Creme, einem Gel oder Hydrogel vor. Das D20 wird durch direkten Kontakt des flüssigen D20 bzw. einer D20-enthaltenden Formulierung von den Epithelzellen des Auges aufgenommen.
4) Für den Fall, dass reines flüssiges D20 allein verabreicht wird (reines D20), ist auszuführen, dass D20 gegenüber allen anderen flüssigen pharmazeutischen Wirkstoffen einen einzigartigen Vorteil hat. Es kann wie normales Wasser (H2O) in die Epithelzellen des Auges transportiert werden und durch die Stärke und Richtung des osmotischen Gradienten und einer Manipulation dieser beiden Größen kann darüber hinaus die Eindringtiefe von D20 in die Epithelzellen an die therapeutische Zielsetzung angepasst werden.
5) Die Wasserstoffbrückenbindungsstärke von Deuteriumatomen ist, wie bereits beschrieben, höher als bei Wasserstoffatomen, insbesondere bei der Bindung von Wasser an organische Moleküle. Topisch verabreichtes D20 bindet molekular durch Wasserstoffbrückenbindungen an die nächste verfügbare Zelloberfläche und verdrängt dabei das dort angelagerte H2O aufgrund seiner höheren Bindungsstärke. Die Austausch-Frequenz der D20-Moleküle mit der H2O-Umgebung ist wiederum durch diese erhöhte Bindungsstärke (und durch das höhere Gewicht des D20-Moleküls) etwas langsamer als für H2O (König, S.,et al. Molecular dynamics of water in oriented multilayers studied by quasi-elastic neutron scattering and deuterium-NMR relaxation. 1994. J.Chem. Phys. 100, 3307-3316). Damit ergibt sich eine erhöhte Aufenthaltswahrscheinlichkeit der D20-Moleküle unmittelbar auf der Zelloberfläche, verbunden mit einer verstärkten Internalisierung von D20 in der Zelle, wodurch es seine Wirkung entfalten kann - die Hemmung oder Inhibierung der oben beschriebenen enzymatischen Reaktionen einer Virus-infizierten Wirtszelle sowie deren Teilung. Da Virus-infizierte Zellen eine höhere Aufnahmefähigkeit für Wasser bzw. D20 aufweisen als normale Zellen, ist darüber hinaus gesichert, dass D20 überproportional in diesen Zellen im Vergleich zu gesunden Zellen angereichert wird, d.h. in einer ausreichenden D20-Konzentration von mehr als 20% bezogen auf den Gesamtwassergehalt der Zelle. Aufgrund dieser Eigenschaften ist die topische Verabreichung von D20 außerordentlich wertvoll.
6) D20 ist das einzige nicht-radioaktive Molekül, welches dem H2O in seinen Eigenschaften sehr ähnlich ist. Zellen allgemein, und insbesondere die erfindungsgemäßen Zellen, können zwischen den beiden Molekülen nicht "unterscheiden", so dass D20 durch aktiven und passiven Transport auf die gleiche Weise wie H2O in die Zelle transportiert wird und bis in die Zellkerne gelangt. Hierdurch werden Zellbarrieren beliebiger Art, die ein Eindringen anderer pharmazeutischer Wirkstoffe verhindern, umgangen und ebenfalls Abwehrmechanismen auf zellulärer Ebene, wie die Internalisierung in Lysosomen oder die Aktivierung von MDR (multiple drug resistance) Transportern oder auf Organebene durch das Immunsystem, welche die Wirksamkeit des pharmazeutischen Wirkstoffs D20 reduzieren oder inhibieren könnten, sind weitgehend ausgeschaltet.
7) Ein weiterer Vorteil von D20 als antiviraler bzw. virostatischer Wirkstoff ist die Tatsache, dass Konzentrationen von weniger als 20% D20 (bezogen auf den Gesamtwassergehalt der Zelle) in der Zelle keine signifikanten Wirkungen entfalten und damit normale Zellen, die wegen ihrer gegenüber den aktiven, Virus-infizierten Zellen geringeren Wasserpermeabilität und/oder Wasseraufnahme vergleichsweise wenig D20 aufnehmen, kaum den Wirkungen des D20 ausgesetzt sind.

In einer offenbarten Ausführungsform der vorliegenden Erfindung wird D20 erfindungsgemäß nicht-systemisch verabreicht.

Eine besonders bevorzugte Ausführungsform der Erfindung ist die Verwendung von Deuteriumoxid zur Prophylaxe und/oder Therapie von Virus-basierten Erkrankungen des Auges, wobei das Deuteriumoxid topisch verabreicht wird. Eine solche topische Verabreichung erfolgt vorzugsweise in Form von Tropfen, d.h. in flüssiger Form, wie beispielsweise als Augentropfen (D2O-Augentropfen) oder einer Spülung (D20-Augenspülung), oder in fester Form als Creme (D20-Creme), Salbe (D20-Salbe), Gel (D20-Gel) oder Hydrogel (D20-Hydrogel). Hierdurch werden vor allem Nebenwirkungen, die durch eine systemische Verabreichung von antiviralen bzw. virostatischen Wirkstoffen verursacht werden, verhindert.

Eine weitere besonders bevorzugte Ausführungsform der Erfindung ist die Verwendung von Deuteriumoxid, wobei das Deuteriumoxid das Auge bzw. die zu behandelnden Organe des Auges, insbesondere die Schleimhäute des Auges, hydratisiert.

Durch eine erfindungsgemäße direkte Verabreichung auf Virus-infizierte Organe des Auges bzw. zu behandelnde Organe des Auges, insbesondere die Schleimhäute des Auges, können lokal prophylaktisch bzw. therapeutisch wirksame D2O-Konzentrationen und eine lokal prophylaktisch bzw. therapeutisch wirksame Hydratation durch D20 erreicht werden und gleichzeitig eine Belastungen des Systems (d.h. des Blutkreislaufs) und die Nebenwirkungen auf nicht zu behandelndes, gesundes Gewebe und Organe des Auges sowie Gewebe von anderen umliegenden oder entfernt liegenden Organen (wie beispielsweise der Leber oder Niere, die durch eine hohe Konzentration von D20 von mehr als 20% D20, bezogen auf den Gesamtwassergehalt der Zelle, verursacht werden könnten), vermindert bzw vollständig vermieden werden. Ein Transport von D20 in das System kann darüber hinaus mit Mitteln, die im Stand der Technik gut bekannt sind, verhindert oder eingeschränkt werden kann. Beispiele für diese Mittel sind u.a. die gezielte Manipulation des osmotischen Gradienten über die Schleimhaut (d.h. zwischen systemischen Teil und der Schleimhautoberfläche) durch Verringerung des Wasserpotentials des verabreichten D20 mittels Substanzen, die geeignet sind, dieses Wasserpotential zu verändern, insbesondere physiologisch verträgliche Salze, wie Natriumchlorid, wasserlösliche Polymere und andere nicht-pharmazeutische Stoffe.

D20 kann erfindungsgemäß allein als pharmazeutischer Wirkstoff, genauer als antiviraler oder virostatischer Wirkstoff, oder in Kombination mit einem oder mehreren weiteren pharmazeutischen Wirkstoff(en) oder mit einem oder mehreren weiteren nicht-pharmazeutischen Wirkstoff(en) (welche insbesondere zur Optimierung der topischen Verabreichung von D20 als pharmazeutischen Wirkstoff auf den Schleimhäuten dienen) verabreicht werden.

Eine bevorzugte Ausführungsform der vorliegenden Erfindung betrifft demnach die Verwendung von Deuteriumoxid zur Prophylaxe und/oder Therapie von Virus-basierten Erkrankungen des Auges, wobei das D20 in Kombination mit mindestens einem weiteren pharmazeutischen Wirkstoff und/oder mindestens einem weiteren nicht-pharmazeutischen Wirkstoff verwendet wird.

Eine solche Kombination aus D20 und mindestens einem weiteren pharmazeutischen Wirkstoff und/oder mindestens einem weiteren nicht-pharmazeutischen Wirkstoff wird nachfolgend als "erfindungsgemäße Kombination" bezeichnet.

Sämtliche in dieser Beschreibung offenbarten erfindungsgemäßen Verwendungen und Verabreichungen, beispielsweise als Formulierung, Flüssigkeit, Hydrogel, Gel, Creme oder Salbe oder in einem Lösungsmittel, topisch etc., von D20 sind ebenfalls auf eine erfindungsgemäße Kombination uneingeschränkt anwendbar, sofern nichts Gegenteiliges angezeigt ist. Gleiches gilt für die Verwendung und Verabreichung von D20 in Kombination mit H2O, auch nachfolgend als "Mischung aus D20 und H2O" bezeichnet.

Eine weiterhin bevorzugte Ausführungsform der vorliegenden Erfindung betrifft die Verwendung von Deuteriumoxid zur Prophylaxe und/oder Therapie von Virus-basierten Erkrankungen des Auges, wobei das D20 in Kombination mit mindestens einem weiteren pharmazeutischen Wirkstoff verwendet wird, wobei dieser ausgewählt ist aus der Gruppe, bestehend aus Virostatika, Proteinen, Peptiden, Nukleinsäuren und immunosuppressiven Wirkstoffen.

Nachfolgend werden erfindungsgemäß bevorzugte weitere pharmazeutische Wirkstoffe einer erfindungsgemäßen Kombination und deren Wirkung aufgeführt, wobei diese Aufzählung nur beispielhaft ist und die vorliegende Erfindung nicht hierauf beschränkt ist:

### Virostatika

Als Virostatika werden Wirkstoffe bezeichnet, die die Vermehrung von Viren hemmen. Virostatika hemmen die Aktivität von Enzymen, beispielsweise DNA-Polymerase, reverse Transkriptase oder Proteasen, und hemmen oder inhibieren somit die Replikation des Virus oder die Prozessierung eines synthetisierten langen Virusproteins in kleinere Proteinabschnitte. Beispiele hierfür sind: Amantadin, Rimantadine.

### Immunsuppressive Wirkstoffe

Durch Zugabe von immunsuppressiven Wirkstoffen, beispielsweise Kortikoiden und/oder anderen oder Immunmodulatoren kann die Reaktion des Epithel- oder Hautgewebes auf die Vermehrung der Viren, insbesondere bei bereits vorhandenen Entzündungen, verbessert und optimiert werden.

### Proteine

Als erfindungsgemäß einsetzbare Proteine sind Proteine zu verstehen, die auf geeignete Weise in den Infektions-, Replikations- oder Vermehrungszyklus des Virus in einer Wirtszelle eingreifen. Auf geeignete Weise bedeutet in diesem Zusammenhang, dass die Proteine die Adsorption des Virus an die Wirtszelle, die Injektion der Virus-Nukleinsäure in die Wirtszelle, die Replikation der DNA der Wirtszelle bzw. der Nukleinsäure des Virus, das Prozessieren der Virus-Nukleinsäure oder den Zusammenbau der Viruspartikel zu einem vollständigen Virus hemmen, bevorzugt inhibieren, oder anderweitig in den Vermehrungszyklus des Virus angreifen. Beispiele hierfür sind Protease-Hemmer, Uncoating-Hemmer, Penetrations-Hemmer, reverse Transkriptions-Hemmer und DNA-Polymerase-Hemmer.

### Peptide

Als erfindungsgemäß einsetzbare Peptide sind beispielsweise Peptide zu verstehen, die auf geeignete Weise die Membranpermeabilität der Wirtszellenmembran beeinflussen, insbesondere erhöhen. Hierdurch kann ein verbesserter Transport von D20 und ggf. der weiteren pharmazeutischen oder der nicht-pharmazeutischen Wirkstoffe der Erfindung in die Wirtszelle erreicht werden. Ein Beispiel hierfür ist Mellitin. Darüber hinaus sollen als erfindungsgemäß einsetzbare Peptide all jene Peptide verstanden werden, welche Wirkungen analog der oben für Proteine beschriebenen aufweisen.

### Nukleinsäuren

Durch Zugabe von Nukleinsäuren kann parallel zur antiviralen oder virostatischen Wirkung von D20 eine Veränderung der Erbinformation der Zellen im Bereich des Wirkortes oder ein gezieltes Ausschalten ("Gene Silencing") bestimmter Gene, beispielsweise der DNA-Polymerase, von Zellen im Bereich des Wirkortes, d.h. des zu behandelnden Organ des Auges, und dadurch eine Modifikation des Proteoms erreicht werden. Das "Gene Silencing" kann beispielsweise dazu führen, dass die in die DNA-Schadenabwehr involvierten Gene (zum Beispiel p53, BRCA1, BRCA2, ATM, CHK2) abgeschaltet werden und damit die durch D20 an der Vermehrung gehinderten Viren in den Zellen auch langfristig (nach Ende der topischen D20-Verabreichung) nicht mehr in ein latentes Stadium zurückkehren, sondern langfristig an der Expression viraler DNA gehindert werden. Methoden zum Durchführen eines "Gen Silencing" sind dem Fachmann gut bekannt und beispielsweise in Mello C C , Conte D "Revealing the world of RNA interference", in Nature 431, 338-342 (16. September 2004) beschrieben. Bevorzugt handelt es sich bei den Nukleinsäuren um DNA, vorzugsweise Oligonukleotide, Sense- oder Antisense-DNA, natürliche oder synthetische, cDNA, genomische DNA, nackte DNA, einzel- oder doppelsträngige DNA, oder zirkuläre DNA, oder um RNA, vorzugsweise Antisense-RNA, RNAi, siRNA, oder andere zur Interferenz geeignete RNA-Moleküle, die in ihrer Länge nicht beschränkt sind.

Die Konzentration von erfindungsgemäß neben D20 als pharmazeutischen Wirkstoff verwendeten weiteren pharmazeutischen Wirkstoffen bezogen auf die Gesamtlösung einer erfindungsgemäßen Kombination, liegt im Bereich von mindestens 10⁻⁸ M bis mindestens 5 • 10⁻² M, bevorzugt von mindestens 10⁻⁷ M bis 10⁻³ M, am stärksten bevorzugt von mindestens 10⁻⁶ M bis mindestens 10⁻² M. Ein insbesondere bevorzugter Konzentrationsbereich liegt im Bereich von mindestens 10⁻⁹ M bis mindestens 10⁻² M.

Eine ebenfalls bevorzugte Ausführungsform der vorliegenden Erfindung betrifft die Verwendung von Deuteriumoxid zur Prophylaxe und/oder Therapie von Virus-basierten Erkrankungen des Auges, wobei das D20 in Kombination mit mindestens einem weiteren pharmazeutischen Wirkstoff verwendet wird, wobei der mindestens eine weitere nicht-pharmazeutische Wirkstoff ausgewählt ist aus der Gruppe, bestehend aus pharmazeutisch verträglichen anorganischen oder organischen Säuren oder Basen, Salzen, Vitaminen, Provitaminen, Polymeren, Copolymeren, Block-Copolymeren, Einfachzucker, Mehrfachzucker, ionischen und nicht-ionischen Tensiden oder Lipiden sowie Mischungen hiervon, Albumin, Transferrin und DNA-Reparatur-Proteinen, wie Kinase-Inhibitoren.

Der Begriff "nicht-pharmazeutischer Wirkstoff" bezeichnet im Sinne der Erfindung jede(s) pharmakologisch verträgliche und therapeutisch sinnvolle Molekül, Substanz oder Verbindung, das/die kein pharmazeutischer Wirkstoff ist, jedoch vorzugsweise zusammen mit mindestens einem erfindungsgemäßen pharmazeutischen Wirkstoff an einen zu behandelnden Organismus verabreicht wird, beispielsweise in einer erfindungsgemäßen Formulierung formuliert, um qualitative Eigenschaften des/der pharmazeutischen Wirkstoffs/Wirkstoffe zu beeinflussen, insbesondere zu verbessern. Bevorzugt entfalten die nicht-pharmazeutischen Wirkstoffe keine oder im Hinblick auf die beabsichtigte Prophylaxe oder Therapie von Virus-basierten Erkrankungen des Auges keine nennenswerte oder zumindest keine unerwünschte pharmakologische Wirkung. Geeignete nicht-pharmazeutische Wirkstoffe sind beispielsweise pharmakologisch unbedenkliche Salze, beispielsweise Natriumchlorid, Geschmackstoffe, Vitamine, z.B. Vitamin A oder Vitamin E, Tocopherole oder ähnliche im menschlichen Organismus vorkommende Vitamine oder Provitamine, Antioxidantien, wie beispielsweise Ascorbinsäure, sowie Stabilisatoren und/oder Konservierungsstoffe zum Verlängern der Verwendungs- und Aufbewahrungsdauer eines pharmazeutischen Wirkstoffes oder einer erfindungsgemäßen Formulierung und sonstige dem Fachmann bekannte, übliche nicht-pharmazeutische Wirkstoffe bzw. Hilfs- und Zusatzstoffe.

Nachfolgend werden erfindungsgemäß bevorzugte weitere nicht-pharmazeutische Wirkstoffe einer erfindungsgemäßen Kombination und deren Wirkung sowie geeignete Konzentrationen aufgeführt, wobei diese Aufzählung nur beispielhaft ist und die vorliegende Erfindung nicht hierauf beschränkt ist:

### Wasserlösliche Hilfs- und Zusatzstoffe

Durch Zugabe von wasserlöslichen Hilfs- und Zusatzstoffen, wie z.B. pharmazeutisch verträglichen anorganischen oder organischen Säuren, Basen, Salzen und/oder Puffersubstanzen zur Einstellung des pH Wertes, kann die physiologische Verträglichkeit von D20 in den Zellen der Organe des Auges für nicht Virus-infizierte Zellen verbessert werden. Beispiele für bevorzugte anorganische Säuren sind ausgewählt aus der Gruppe, bestehend aus Salzsäure, Bromwasserstoffsäure, Salpetersäure, Schwefelsäure und Phosphorsäure, wobei insbesondere Salzsäure und Schwefelsäure bevorzugt sind. Beispiele für besonders geeignete organische Säuren sind ausgewählt aus der Gruppe bestehend aus Äpfelsäure, Weinsäure, Maleinsäure, Bernsteinsäure, Essigsäure, Ameisensäure und Propionsäure und insbesondere bevorzugt Ascorbinsäure, Fumarsäure und Zitronensäure. Gegebenenfalls können auch Mischungen der genannten Säuren eingesetzt werden, insbesondere von Säuren, die neben ihren Säuerungseigenschaften auch andere Eigenschaften, z.B. in Verwendung als Antioxidantien, besitzen, wie beispielsweise Zitronensäure oder Ascorbinsäure. Beispiele für pharmazeutisch verträgliche Basen sind Alkalihydroxide, Alkalicarbonate und Alkali-Ionen, bevorzugt Natrium. Mischungen dieser Substanzen können insbesondere zur Einstellung und Pufferung des pH Wertes benutzt werden, besonders bevorzugt sind hierbei Kaliumhydrogenphosphat und Di-Kaliumhydrogenphosphat. Natriumhydrogenphosphat und Di-Natriumhydrogenphosphat sowie Zitronensäure und Natriumcitrat. Bevorzugte Puffersubstanzen im Sinne der Erfindung sind weiterhin PBS, HEPES, TRIS, MOPS sowie weitere physiologisch verträgliche Puffersubstanzen, insbesondere jene mit einem pK Wert im Bereich 4,0 bis 9,0. Die Konzentration dieser Substanzen, bezogen auf die Gesamtlösung einer erfindungsgemäßen Kombination, liegt vorzugsweise im Bereich von Mikromolar bis Millimolar, besonders bevorzugt im Bereich 1-100 mM.

### Wasserlösliche, polymere Moleküle

Durch Zugabe von wasserlöslichen, nicht-zytotoxischen Molekülen, wie z.B. bestimmten Polymeren (z.B., aber nicht hierauf beschränkt, Dextran, Polyethylenglykol, Agarose, Zellulose, Acrylsäre, Hylaronsäure), Copolymeren und Block-Copolymeren kann durch deren hohe Wasserbindungskapazität eine zusätzliche Verzögerung (Retardierung) des Übergangs des D20 bei topischer Verabreichung in die Epithelzellen des Auges wie auch von den Epithelzellen ins System (Blutkreislauf), erreicht werden. Durch die Fähigkeit der Polymere, das chemische Potential (Wasserpotential) von D20 zu erniedrigen, kann darüber hinaus die Stärke und Richtung des osmotischen Gradienten über die Haut verändert bzw. verbessert und optimiert werden. Die Konzentration dieser Substanzen, bezogen auf die Gesamtlösung, liegt im Bereich von Mikromolar bis Molar, bevorzugt im Bereich 1- 500 mM.

### Wasserlösliche, nicht-polymere Moleküle

Durch Zusatz von wasserlöslichen, nicht-polymeren Molekülen, welche die Dichte und/oder Viskosität von D20 verändern, beispielsweise, aber nicht hierauf beschränkt, Einfachzucker und Mehrfachzucker, insbesondere Glucose, Sacharose, Dextrose, Maltose, Stärke und Cellulose, können die osmotischen Verhältnisse im Bereich der topischen D20 Verabreichung sowie der D2O-Transport und die D2O-Retention in die Epithelzellen des Auges verändert bzw. optimiert werden. Die Konzentration dieser Substanzen, bezogen auf die Gesamtlösung einer erfindungsgemäßen Kombination, liegt vorzugsweise im Bereich von Millimolar bis Molar, besonders bevorzugt im Bereich 1,0 mM bis 1,5 M.

### D2O-Grenzflächenspannungs-verändernde Moleküle

Durch Zugabe von Substanzen, welche die Grenzflächenspannung von D20 verändern, beispielsweise, aber nicht hierauf beschränkt, ionische und nicht-ionische Tensiden oder Lipide, insbesondere einer Mischung aus Tensiden und Lipiden, kann der Transport des D20 bei topischer Verabreichung in die Epithelzellen des Auges sowie innerhalb dieser Epithelzellen verändert werden. Die Konzentration dieser Substanzen, bezogen auf die Gesamtlösung einer erfindungsgemäßen Kombination, liegt vorzugsweise im Bereich von Mikomolar bis Millimolar, besonders bevorzugt im Bereich 0,05 - 500 mM.

### Wasserlösliche nicht-cytotoxische Moleküle

Durch Zugabe von wasserlöslichen Molekülen, welche dafür bekannt sind, dass sie durch metabolisch besonders aktive Zellen, wie z. B. Virus-infizierte Zellen, in besonderem Maße aufgenommen werden, beispielsweise Albumin oder Transferrin, kann eine zusätzliche Erhöhung der D2O-Transportrate der von einer D20-Hydrathülle umgebenen Molekülen in die Targetzellen des Auges erreicht werden.

Die Konzentration bzw. Dosierung von D20 und ggf. des mindestens einen weiteren pharmazeutischen und/oder nicht-pharmazeutischen Wirkstoffs unterliegt verschiedenen Faktoren, beispielsweise der Art der Behandlung, Art der Erkrankung, Erkrankungszustand des Patienten (Säugetier), Art des Wirkstoffs usw.. Dem Fachmann sind solche Parameter bekannt und die Bestimmung der speziellen Dosierungen unterliegt dem Fachwissen des Fachmanns. Geeignete Konzentrationsangaben werden hierin offenbart. Einige beispielhafte Angaben zu geeigneten Konzentrationsbereichen sind bereits oben aufgeführt, wobei diese lediglich Richtwerte darstellen sollen.

Das erfindungsgemäß verwendbare D20 liegt bevorzugt als Flüssigkeit vor. Vorzugsweise liegt das D20 in einer Lösung, bevorzugt mit H2O (Wasser) als Lösungsmittel, vor und wird hierin auch als "Mischung aus D20 und H2O" oder "D2O/H2O", wenn H2O enthalten ist, bezeichnet, bzw. als "D2O-Lösung" oder "reines D20", wenn kein H2O enthalten ist, bezeichnet. Reines D20 enthält D20 vorzugsweise in einem Konzentrationsbereich von 98,0 bis 100%, bevorzugt von 98,5 bis 99,9%, besonders bevorzugt von 99,7% bezogen auf den Gesamtwassergehalt der Lösung. Eine erfindungsgemäße Mischung aus D20 und H2O enthält D20 vorzugsweise in einem Konzentrationsbereich von 1 bis 99%, bevorzugt von 5 bis 95%, weiterhin bevorzugt von 10 bis 90%, ebenfalls bevorzugt von 15 bis 80%, stärker bevorzugt von 20 bis 70%, ebenfalls stärker bevorzugt von 30 bis 60%, am stärksten bevorzugt von 40 bis 50%, wobei sich diese Angaben auf den Gesamtwassergehalt der Mischung aus D20 und H2O beziehen. Die Herstellung einer erfindungsgemäßen D20-Lösung und analog hierzu einer erfindungsgemäßen Kombination erfolgt beispielsweise durch Mischen der Komponenten, insbesondere von D20 und ggf. H2O sowie ggf. des mindestens einen weiteren pharmazeutischen und/oder nicht-pharmazeutischen Wirkstoffs. Weiterhin kann ggf. ein Lösungsmittel, wie nachfolgend beschrieben, durch Mischen hinzugefügt werden. Vorzugsweise erfolgt die Beimischung von H2O bzw. des mindestens einen weiteren pharmazeutischen und/oder nicht-pharmazeutischen Wirkstoffs bzw. des Lösungsmittels zu D20 im flüssigen Aggregatzustand. Die Herstellung kann jedoch durch jedes beliebige geeignete Verfahren erreicht werden. Im Fall, dass D20 allein (d.h. nicht in Kombination mit H2O oder mindestens einem weiteren pharmazeutischen und/oder nicht-pharmazeutischen Wirkstoff) und bei einer Konzentration von 100% bzw. ca. 99.9% bezogen auf das Gesamtvolumen der Lösung (d.h. reines D20) verwendet wird, stellt reines D20 die erfindungsgemäße D20-Lösung dar.

Sämtliche in dieser Beschreibung offenbarten erfindungsgemäßen Verwendungen und Verabreichungen, beispielsweise als Formulierung, Flüssigkeit, Hydrogel, Gel, Creme oder Salbe oder in einem Lösungsmittel, topisch etc., von D20 sind ebenfalls auf eine erfindungsgemäße Mischung aus D20 und H2O uneingeschränkt anwendbar, sofern nichts Gegenteiliges angezeigt ist. Ebenfalls finden Verwendungen und Verabreichungen einer erfindungsgemäßen Kombination auf eine erfindungsgemäße Mischung aus D20 und H2O uneingeschränkt Anwendung und umgekehrt sofern nichts Gegenteiliges angezeigt ist.

In einer offenbarten Ausführungsform ist der mindestens eine weitere pharmazeutische Wirkstoff bzw. weitere nicht-pharmazeutische Wirkstoff an D20 gebunden. "Gebunden" im Sinn der vorliegenden Erfindung bedeutet, dass der pharmazeutische bzw. nicht-pharmazeutische Wirkstoff von dem D20 hydratisiert wird.

In weiteren offenbarten Ausführungsformen ist das D20 bzw. die erfindungsgemäße Kombination in einem geeigneten Lösungsmittel enthalten. Ein erfindungsgemäßes Lösungsmittel kann ein anorganisches oder organisches Lösungsmittel sein. Geeignete Lösungsmittel der vorliegenden Erfindung sollten vorzugsweise von dem Organismus (insbesondere Säugetier), dem der Wirkstoff mit Lösungsmittel verabreicht wird, physiologisch gut verträglich sein, d.h. keine Nebenwirkungen, z.B. toxische Nebenwirkungen, auslösen. Ein insbesondere bevorzugtes Lösungsmittel ist destilliert es Wasser. Ebenfalls bevorzugt sind Ethanol-Wasser-Mischungen; hierbei liegt der prozentuale Massenanteil von Ethanol in diesen Mischungen bevorzugt im Bereich zwischen 5 % und 99 % Ethanol, ebenfalls bevorzugt im Bereich von 10 % bis 96 % Ethanol, stärker bevorzugt zwischen 50 % und 92 %, am stärksten bevorzugt zwischen 69 % und 91 % Ethanol.

D20 oder eine erfindungsgemäße Kombination oder eine erfindungsgemäße Mischung aus D20 und H2O können "vorformuliert" vorliegen, beispielsweise verpackt in geeigneten Mitteln zum Transport von pharmazeutischen Wirkstoffen, sog. "drug delivery"-Systemen, beispielsweise in Nanopartikeln, Vektoren, bevorzugt Gentransfer-Vektoren, viralen oder nicht viralen Vektoren, Poly- oder Lipoplex-Vektoren, Liposomen oder Hohlkolloide (d.h. Hohlkugeln kolloider Dimension). Weiterhin zum Transport geeignet sind nackte Nukleinsäuren, insbesondere nackte DNA. Geeigneten Vektoren, Liposomen, Hohlkolloide oder Nanopartikel sowie Verfahren zum Einbringen von Substanzen, in solche Vektoren, Liposomen, Hohlkolloide oder Nanopartikel sind allgemein im Stand der Technik gut bekannt und beispielsweise in Cryan S-A., Carrier-based strategies for Targeting Protein and Peptide Drugs to the Lungs, AAPS Journal, 2005, 07(01):E20-E41 und Sambrook et al., Molecular Cloning A Laboratory Manual, Cold Spring Harbor Laboratory (1989) NY beschrieben. Als Gentransfer-Vektoren können vorzugsweise polyethylenimine oder kationische Lipide, wie z.B DOTAP, verwendet werden. Liposomen sind bevorzugt für die Verpackung von Zytostatika verwendbar; eine detaillierte Beschreibung erfolgt beispielsweise in Koshkina NV et al., Koshkina, N.V., et al., Paclitacel liposome aerosol treatment induces inhibition of pulmonary metastases in murine renal carcinoma model., Clinical Cancer Research, 2001, 7, 3258-3262. Proteine als pharmazeutische Wirkstoffe können vorzugsweise mittels superkritischer Flüssigkeiten, Emulsionsverfahren und Sprühtrocknung in biokompatible Poly-Milch/Glykolsäure-Polymere (PLGA) verpackt werden.

D20 wird erfindungsgemäß bevorzugt topisch verabreicht. Dies erfolgt durch Auftragung, Aufbringung bzw. Einbringung von D20 auf/in die Epithelzellen bzw. Schleimhäute des zu behandelnden Organs des Auges vorzugsweise in Form von Tropfen, d.h. in flüssiger Form, wie beispielsweise als Tropfen Spülung, oder in fester Form, beispielsweise als Creme, Salbe, Gel oder Hydrogel, oder in Form einer Formulierung,. Die Art und Dauer der topischen Verabreichung von D20 zur Prophylaxe und/oder Therapie von Virus-basierten Erkrankungen des Auges sowie die Konzentration des D20 sowie ggf. weiterer pharmazeutischer und/oder nicht pharmazeutischer Wirkstoffe ist neben der Schwere der Erkrankung und des Zustandes des Patients von der Lokalisation und Zugänglichkeit des zu behandelnden Organs des Auges abhängig.

Eine bevorzugte Ausführungsform der Erfindung betrifft die Verwendung von Deuteriumoxid zur Prophylaxe und/oder Therapie von Virus-basierten Erkrankungen des Auges, wobei das D20 als Formulierung verabreicht wird. Die Formulierung wird topisch verabreicht, indem es auf das zu behandelnde Organ des Auges, insbesondere auf dessen Epitelzellen oder Schleimhaut aufgetragen wird. Besonders bevorzugt ist eine erfindungsgemäße Formulierung eine Flüssigkeit, Creme, Salbe, Gel oder ein Hydrogel ist. Vor allem bevorzugt ist eine erfindungsgemäße Forumulierung eine Augenspülung (D20-Augenspülung), Augentropfen (D2O-Augentropfen), eine Augensalbe (D20-Augensalbe), ein Augengel (D20-Augengel) oder ein Augenhydrogel (D20-Augenhydrogel).

Eine solche "Flüssigkeit" oder auch flüssige Formulierung im Sinne der Erfindung umfaßt Formulierungen aus reinem D20, aus einer Mischung aus D20 und H2O und aus einer erfindungsgemäßen Kombination, soweit diese in flüssigem Zustand vorliegt. Sie wird vorzugsweise in Form von Tropfen oder als Spülung verabreicht.

Erfindungsgemäße Formulierungen, auch D20-Formulierungen sind zur Behandlung von Virus-basierten Erkrankungen des Auges geeignet. Sie werden auf das zu behandelnde Organ, insbesondere auf dessen Epithelzellen oder Schleimhäute topisch aufgetragen. Sie werden hierzu vorzugsweise als Flüssigkeit, bevorzugt als Spülung oder Tropfen, oder als Creme, Salbe, Gel oder Hydrogel verabreicht:
- D20-Spülungen werden zum Spülen des Auges eingesetzt. Auf diese Weise kann D20 topisch und in hohen Konzentrationen auf das zu behandelnde Organ, insbesondere dessen Epithelzellen oder Schleimhäute, auf- und eingebracht werden. Eine D20-Spülung ist beispielsweise besonders geeignet für die Behandlung von Keratokonjunktivitis sicca.
- D2O-Augentropfen können zur direkten lokalen Verabreichung des D20 auf das zu behandelnde Organ, insbesondere dessen Epithelzellen oder Schleimhäute, eingesetzt werden. D2O-Augentropfen werden hierzu in das Auge getropft. D2O-Augentropfen sind beispielsweise besonders geeignet für die Behandlung von Keratokonjunktivitis sicca.
- D20-Creme, D20-Salbe, D20-Gel und D20-Hydrogel werden topisch auf das zu behandelnde Organ, insbesondere dessen Epithelzellen oder Schleimhäute auf- und eingebracht.

Unter einer "Salbe" gemäß der vorliegenden Erfindung ist eine äußerlich zu verwendende Arzneimittelzubereitung aus einer Grundmasse schmierfähiger Stoffe, wie Vaseline, zu der die eigentlichen pharmazeutischen Wirkstoffe, wie D20, und/oder nicht pharmazeutische Wirkstoffe zugegeben werden, beispielsweise durch Mischen.

Unter einer "Creme" im Sinne der vorliegenden Erfindung ist eine erfindungsgemäße Salbe zu verstehen, die zusätzlich weitere Bestandteile enthalten kann, wie kosmetische Wirkstoffe, z.B. Duftstoffe, Farbstoffe und/oder Emulgatoren, z.B. Lecithin. Von einer Creme wird im allgemeinen eine Lotion unterschieden, wobei diese Unterscheidung meistens abhängig von dem Grad der Viskosität gemacht wird. Erfindungsgemäß ist unter einer Creme auch eine Lotion zu verstehen.

Ein "Gel" im Sinne der vorliegenden Erfindung ist die Lösung einer makromolekularen Substanz, z. B. Agarose, Acrylsäure, Alginsäure, Polysiloxane oder Acrylamid, deren Konzentration so hoch ist, dass sich die gelösten Makromoleküle unter geeigneten Bedingungen und ggf. unter Zugabe weiterer Substanzen (z.B. Salze, Säuren, Füllstoffe, Puffersubstanzen) zu einem schwammartigen, dreidimensionalen Gerüst verbinden, in dessen Hohlräumen sich eine Flüssigkeit befindet. Dadurch haben Gele eine relativ feste Konsistenz. Die Viskosität liegt zwischen flüssig und fest. Eine solche Flüssigkeit ist bevorzugt reines D20 oder eine erfindungsgemäße Mischung aus D20 und H2O.

Als "Hydrogel" im Sinne der Erfindung wird ein Gel bezeichnet, welches durch besonders hohe Aufnahmekapazität von Wasser gekennzeichnet ist, im Sinne der Erfindung besteht es bevorzugt zu 20-99%, stärker bevorzugt zu 70-99%, und besonders bevorzugt zu 80-99%, aus Wasser, ohne jedoch die rheologischen Eigenschaften einer klassischen Flüssigkeit zu zeigen. In einer besonders bevorzugten Ausführungsform ist das Hydrogel transparentdurchsichtig und gleichzeitig streichfähig, ohne das seine Morphologie und Integrität durch das verstreichen des Gels beeinträchtigt wird.

Die Herstellung einer erfindungsgemäß verwendbaren Formulierung, insbesondere einer Salbe, Creme oder einem Gel, ist exemplarisch in den Beispielen beschrieben. Sofern eine solche Formulierung weitere pharmazeutische und/oder nicht-pharmazeutische Wirkstoffe enthalten, werden diese vorzugsweise durch Mischen der Formulierung hinzugefügt. Sie kann jedoch nach jedem beliebigen im Stand der Technik bekannten Standardverfahren erfolgen. Dem Fachmann sind solche Verfahren und ebenfalls die zu wählenden Konzentrationen der zu verwendenden Komponenten bzw. Substanzen bekannt.

Die Konzentrationen von D20 in einer erfindungsgemäß verwendbaren Formulierung liegen vorzugsweise in folgenden Bereichen:
- für eine Creme oder Salbe bevorzugt im Bereich von 0,1 bis 98 Gew.-%, bevorzugt von 5 bis 85 Gew.-%, ebenfalls bevorzugt von 10 bis 80 Gew.-%, besonders bevorzugt von 15 bis 70 Gew.-%, stärker bevorzugt von 20 bis 60 Gew.-% und am stärksten bevorzugt von 25 bis 50 Gew.-% und
- für ein Gel bevorzugt 0,1 bis 99,8 Gew.-%, bevorzugt von 10 bis 99 Gew.-%, ebenfalls bevorzugt von 15 bis 80 Gew.-%, besonders bevorzugt von 20 bis 70 Gew.-%, stärker bevorzugt von 30 bis 70 Gew.-% und am stärksten bevorzugt von 35 bis 65 Gew.-%.

Der Fachmann wird insbesondere abhängig von der vorliegenden Indikation, dem Zustand des zu behandelnden Organismus (Patienten), der Schwere der Erkrankung usw. die geeignete Konzentration wählen.

In einer offenbarten Ausführungsform enthält eine erfindungsgemäß verwendbare Formulierung weiterhin mindestens ein anorganisches oder organisches Lösungsmittel. Bevorzugt ist das Lösungsmittel ausgewählt ist aus der Gruppe, bestehend aus Ethanol, Wasser und Glycerol sowie Mischungen davon.

Sämtliche in dieser Beschreibung offenbarten erfindungsgemäßen Verwendungen sind ebenfalls auf eine erfindungsgemäße Formulierung uneingeschränkt anwendbar, sofern nichts Gegenteiliges angezeigt ist. Ebenfalls finden Verwendungen und Verabreichungen einer erfindungsgemäßen Kombination oder einer erfindungsgemäßen Mischung aus D20 und H2O auf eine erfindungsgemäße Formulierung uneingeschränkt Anwendung und umgekehrt sofern nichts Gegenteiliges angezeigt ist.

Die vorliegende Erfindung wird anhand von Figuren und nachfolgenden Beispielen weiter erläutert, wobei diese nur der Illustration dienen und die Gegenstände der Erfindung nicht beschränken.

### Figuren

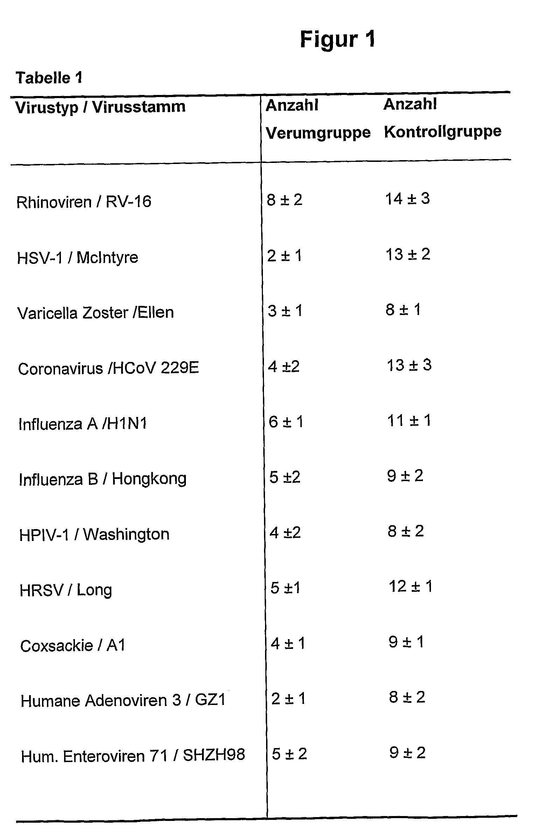

**Figur 1** zeigt **Tabelle 1,** die wiederum die Anzahl der Viren in einer Typ-1 Zellkultur der Zelllinie SV-40 (Humane Korneaepithel-Zelllinie), hergestellt nach Beispiel 4, zum Zeitpunkt 72 Stunden nach der Infektion für verschiedene Viren. Die Bestimmung der Virenanzahl erfolgte mittels Elektronenmikroskopie durch Zählung und Mittelwertbildung aus jeweils 20 nicht lysierten Zellen der Verumgruppe und der Kontrollgruppe.

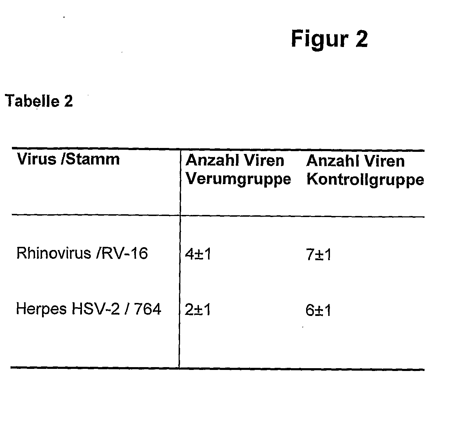

**Figur 2** zeigt **Tabelle 2,** die wiederum die Anzahl der Viren in einer modifizierten Typ-1 Zellkultur der Zelllinie SV-40 (Humane Korneaepithel-Zelllinie), hergestellt nach Beispiel 5, zum Zeitpunkt 72 Stunden nach der Infektion für verschiedene Viren. Die Bestimmung der Virenanzahl erfolgte mittels Elektronenmikroskopie durch Zählung und Mittelwertbildung aus jeweils 20 nicht lysierten Zellen der Verumgruppe und der Kontrollgruppe.

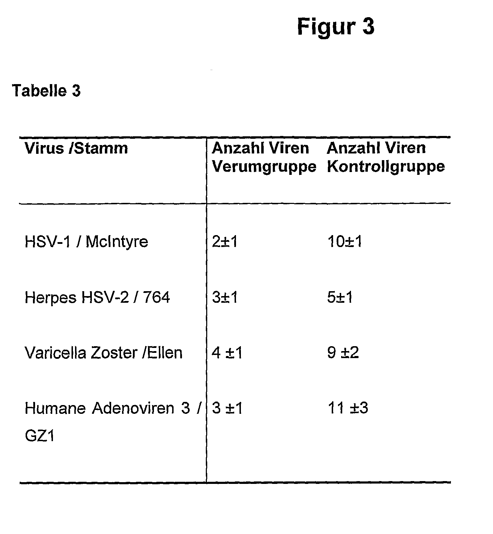

**Figur 3** zeigt **Tabelle 3,** die wiederum die Anzahl der Viren in einer Typ-2 Zellkultur der Zelllinie CCL 20.2 (Humane Konjunktivaepithel-Zelllinie), hergestellt nach Beispiel 6, zum Zeitpunkt 72 Stunden nach der Infektion für verschiedene Viren. Die Bestimmung der Virenanzahl erfolgte mittels Elektronenmikroskopie durch Zählung und Mittelwertbildung aus jeweils 20 nicht lysierten Zellen der Verumgruppe und der Kontrollgruppe.

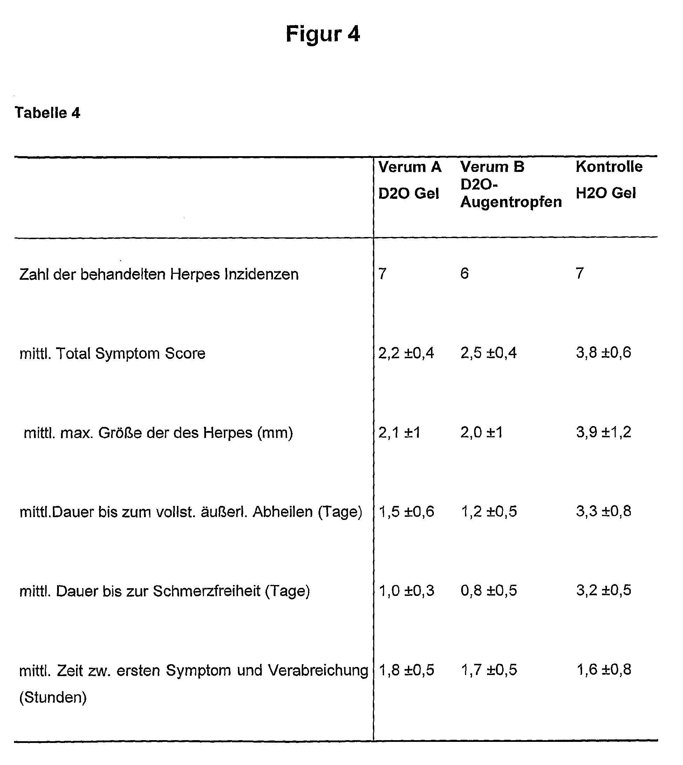

**Figur 4** zeigt **Tabelle 4,** die wiederum die Ergebnisse der Wirksamkeit von D20 Hydrogel (Verum A) und D20 Augentropfen (Verum B) sowie von H2O Hydrogel (Kontrolle) zur Therapie von Augenherpes am Menschen nach Beipiel 25. Die Mittelwertbildung des "Total Symptom Score" erfolgte über die Zahl der Verabreichungen pro Herpes-Inzidenz, bei "maximale Größe des Herpes", "Zeit zwischen ersten Symptom und erster Verabreichung", "Dauer bis zum äußerlich vollständigen Abheilen", "Dauer bis zur Schmerzfreiheit" wurde über die Gesamtzahl der pro Gruppe (Verum bzw. Kontrolle) behandelten Herpes-Inzidenzen gemittelt. Die angegebenen Fehler sind die Standardabweichungen.

### Beispiele

### Beispiel 1: Herstellung einer isotonen D20- bzw. H2O-Lösung zur Spülung oder als Augentropfen

Deuteriumoxid (D20) mit einer Anreicherung von 98% oder gereinigtes Wasser (H2O) wurde mit 160 mM NaCl gemischt und mittels 20 mM Phosphatpuffer (Natrium-dihydrogenphosphat und Di-natrium-hydrogenphosphat) auf einen pH Wert von 7,0 eingestellt. Die Lösungen wurden nach Verfahren, die Stand der Technik sind, sterilisiert und bis zur Verwendung steril und lichtgeschützt gelagert.

### Beispiel 2: Herstellung eines D20- bzw. H2O-Hydrogels auf Basis von Acrylsäure

Es wurde 2,0 Gew.-% Carbopol 980 (Hersteller: Noveon, Inc., 9911 Brecksville Rd., Cleveland, OH 44141-3247, USA) in getrennten Ansätzen in reinem D20 (98% D-Anreicherung), oder in reinem H2O durch Rühren gelöst, anschließend durch Pipettieren von 10 M NaOH Lösung auf einen pH Wert von 6,8 titriert und mittels 20 mM Phosphatpuffer (Natrium-dihydrogenphosphat und Di-natrium-hydrogenphosphat) auf diesen pH Wert gepuffert. Anschließend wurden die durch die NaOH Zugabe infolge Quervernetzung der Polyacrylsäure über ihre Carboxylgruppen mit den alkalischen Hydroxyl-Gruppen entstandenen farblosen, transparenten und optisch klaren Acrylsäuregelen (Carbopolgele) (D20-Carbopolgel, H2O-Carbopolgel) sterilisiert (autoklaviert) und bei Raumtemperatur bis zur weiteren Verwendung, aber für mindestens 24 Stunden, steril abgepackt gelagert.

### Beispiel 3: Herstellung einer D20-haltigen Creme

Zu 50 g Asche Basiscreme (Hersteller: Asche Chiesi GmbH, Hamburg, Deutschland) wurde bei 40 °C unter ständigen Rühren langsam D20 hinzugegeben, bis ein Gewichtsanteil vom 38% D20 (bezogen auf das Ausgangsgewicht der Creme) in der homogenen Mischung erreicht war. Die Creme wurde anschließend auf Raumtemperatur abgekühlt und luftdicht verschlossen gelagert.

### Beispiel 4: Zellkultur Typ 1

Zellen der SV-40 Zelllinie (Humane Korneaepithel-Zelline) pärpariert nach Araki-Sasaki K. et al. ("An SV40-Immortalized Human Corneal Epithelial Cell Line and Its Characterization", Investigative Ophthalmology & Visual Science, March 1995, Vol. 36, No. 3) wurden in 10 mm Zellkulturschalen bei einer Zelldichte von of 8 × 10⁴ Zellen/cm² gesät. Das hierzu verwendete Medium war Dulbecco's Modified Eagle Medium, DMEM, (Gibco/BRL, Life Technologies Inc., Grand Island, NY, USA) mit 10% Fetalen Kälberserum, FCS; (Hyclone, Logan, UT, USA), 100 U/ml Penicillin, and 100 µg/ml Streptomycin (Sigma Chemical Co., St. Louis, MO, USA). Bei einer Temperatur von 37°C wurden die Zellen bei 95% Luftfeuchtigkeit und 5% CO₂ bis zur Konfluenz gezüchtet. Am Tag der Infektion der Zellkultur mit Viren wurde das Zellkulturmedium gewechselt und das neue Medium (DMEM mit 2% FCS und 100 U/ml Penicillin sowie 100 µg/ml Streptomycin) enthielt die Viren in einer Konzentration, die einer Infektionsmultiplizität (MOI) von 3 entsprach. Die Wahrscheinlichkeit, dass eine Zelle mit mindestens einem Viruspartikel infiziert wird, lag bei dieser MOI bei 95%. Die Infektion und Inkubation erfolgte bei Temperatur und Luftfeuchtigkeit analog wie oben angegeben.

### Beispiel 5: Modifizierte Zellkultur Typ 1

Zellen der SV-40 Zelllinie (Humane Corneaepithelzellline) pärpariert nach Araki-Sasaki K. et al. ("An SV40-Immortalized Human Corneal Epithelial Cell Line and Its Characterization" , Investigative Ophthalmology & Visual Science, March 1995, Vol. 36, No. 3) wurden in 10 mm Zellkulturschalen bei einer Zelldichte von of 8 × 10⁴ Zellen/cm² gesät. Das hierzu verwendete Medium war Dulbecco's Modified Eagle Medium, DMEM, (Gibco/BRL, Life Technologies Inc., Grand Island, NY, USA) mit 10% Fetalen Kälberserum, FCS; (Hyclone, Logan, UT, USA), 100 U/ml Penicillin, and 100 µg/ml Streptomycin (Sigma Chemical Co., St. Louis, MO, USA). Bei einer Temperatur von 37°C wurden die Zellen bei 95% Luftfeuchtigkeit und 5% CO₂ bis zur Konfluenz gezüchtet. Nach Erreichen der Konfluenz und 24 Stunden vor der Infektion der Zellkultur mit Viren wurde das Zellkulturmedium gewechselt und das neue Medium (DMEM mit 2% FCS und 100 U/ml Penicillin sowie 100 µg/ml Streptomycin), verdünnt mit 30% D20 , 98% D-Anreicherung (Verumkultur) oder verdünnt mit 30% H2O (Kontrollkultur). Zum Zeitpunkt der Infektion wurde das Medium nochmals gegen ein Medium identischer Zusammensetzung ausgetauscht, welches die Viren in Konzentrationen enthielt, die einer Infektionsmultiplizität von 3 entspricht. Die Infektion und Inkubation erfolgte bei Temperatur und Luftfeuchtigkeit analog wie oben angegeben.

### Beispiel 6: Zellkultur Typ 2

Zellen der CCL 20.2 Zelllinie (Humane Konjuktivaepithel-Zelline) von American Type Culture Collection (Manassas, VA, USA) wurden in 10 mm Zellkulturschalen bei einer Zelldichte von of 8 × 10⁴ Zellen/cm² gesät. Das hierzu verwendete Medium war Dulbecco's Modified Eagle Medium, DMEM, (Gibco/BRL, Life Technologies Inc., Grand Island, NY, USA) mit 10% Fetalen Kälberserum, FCS (Hyclone, Logan, UT, USA), 100 U/ml Penicillin, and 100 µg/ml Streptomycin (Sigma Chemical Co., St. Louis, MO, USA). Bei einer Temperatur von 37°C wurden die Zellen bei 95% Luftfeuchtigkeit und 5% CO₂ bis zur Konfluenz gezüchtet. Am Tag der Infektion der Zellkultur mit Viren wurde das Zellkulturmedium gewechselt und das neue Medium (DMEM mit 2% FCS und 100 U/ml Penicillin sowie 100 µg/ml Streptomycin) enthielt die Viren in einer Konzentration, die einer Infektionsmultiplizität (MOI) von 3 entspricht. Die Wahrscheinlichkeit, dass eine Zelle mit mindestens einem Viruspartikel infiziert wird, lag bei dieser MOI bei 95%. Die Infektion und Inkubation erfolgte bei Temperatur und Luftfeuchtigkeit analog wie oben angegeben.

### Beispiel 7: Bestimmung der Virusanzahl in Zellkultur nach Inkubation

Nach einer Inkubationszeit von 3 Tagen wurden Verumkultur und Kontrollkultur bezüglich der mittleren Anzahl von Viren pro Zelle untersucht. Die Viruszählung erfolgte mittels Transmissions-Elektronenmikroskopie nach Standard Fixier- und Färbeverfahren (Glutaraldehyd und Osmiumtetroxid). Vor der Fixation wurde das Zellkulturmedium entfernt und der Überstand (nach Zentrifugation bei 20.000 x g für 30 Minuten) für weitere Analysen aufbewahrt. Mittels Elektronenmikroskopie wurden jeweils 20 willkürlich ausgewählte, nicht lysierte Zellen aus Verum- und Kontrollkultur bezüglich der in ihnen enthaltenen Viruspartikel ausgewertet.

### Referenz-Beispiel 8: D20-Wirksamkeit bei Herpesviren HSV-1 in Typ-1 Zellkultur

Es wurden 6 Zellkulturschalen der Zellkultur Typ 1 nach Beispiel 4 mit Herpesviren vom Typ 1, HSV-1 (Stamm Maclntyre VR-539 von American Type Culture Collection , Manassas, VA, USA) infiziert. 120 Minuten nach der Infektion wurde das mit den Viren versetzte Zellkulturmedium entfernt und wie folgt durch ein verdünntes Zellkulturmedium ersetzt: Bei der Verumgruppe (3 Zellkulturschalen) durch 30% D20 und 70% DMEM mit 10% FCS und 100 U/ml Penicillin sowie 100 µg/ml Streptomycin. Bei der Kontrollgruppe (3 Zellkulturschalen) wurde für die Verdünnung des Zellkulturmediums anstelle des D20 die gleiche Menge H2O verwendet.

Die Bestimmung der Virusanzahl nach Beispiel 7 für Verum- und Kontrollkultur ergab Werte, die in Tabelle 1 dargestellt sind.

### Referenz-Beispiel 9: D20-Wirksamkeit bei Herpesviren Typ Varicella zoster 1 in Typ-1 Zellkultur

Es wurden 6 Zellkulturschalen der Zellkultur Typ 1 nach Beispiel 4 mit Herpesviren vom Typ Zoster, Varicella Zoster, (Stamm Ellen, VR-586 von American Type Culture Collection, Manassas, VA, USA) infiziert. 120 Minuten nach der Infektion wurde das mit den Viren versetzte Zellkulturmedium entfernt und wie folgt durch ein verdünntes Zellkulturmedium ersetzt: Bei der Verumgruppe (3 Zellkulturschalen) durch 30% D20 und 70% DMEM mit 10% FCS und 100 U/ml Penicillin sowie 100 µg/ml Streptomycin. Bei der Kontrollgruppe (3 Zellkulturschalen) wurde für die Verdünnung des Zellkulturmediums anstelle des D20 die gleiche Menge H2O verwendet.

Die Bestimmung der Virusanzahl nach Beispiel 7 für Verum- und Kontrollkultur ergab Werte, die in Tabelle 1 dargestellt sind.

### Beispiel 10: D20-Wirksamkeit bei Coronaviren Typ Humanes Coronavirus HCoV 229E 1 in Typ-1 Zellkultur

Es wurden 6 Zellkulturschalen der Zellkultur Typ 1 nach Beispiel 4 mit Humanen Coronaviren 229E infiziert. 120 Minuten nach der Infektion wurde das mit den Viren versetzte Zellkulturmedium entfernt und wie folgt durch ein verdünntes Zellkulturmedium ersetzt: Bei der Verumgruppe (3 Zellkulturschalen) durch 30% D20 und 70% DMEM mit 10% FCS und 100 U/ml Penicillin sowie 100 µg/ml Streptomycin. Bei der Kontrollgruppe (3 Zellkulturschalen) wurde für die Verdünnung des Zellkulturmediums anstelle des D20 die gleiche Menge H2O verwendet.

Die Bestimmung der Virusanzahl nach Beispiel 7 für Verum- und Kontrollkultur ergab Werte, die in Tabelle 1 dargestellt sind.

### Refrenz-Beispiel 11: D20-Wirksamkeit bei Influenzavirus A 1 in Typ-1 Zellkultur

Es wurden 6 Zellkulturschalen der Zellkultur Typ 1 nach Beispiel 4 mit Influenzavirus A, Typ H1N1 infiziert. 120 Minuten nach der Infektion wurde das mit den Viren versetzte Zellkulturmedium entfernt und wie folgt durch ein verdünntes Zellkulturmedium ersetzt: Bei der Verumgruppe (3 Zellkulturschalen) durch 30% D20 und 70% DMEM mit 10% FCS und 100 U/ml Penicillin sowie 100 µg/ml Streptomycin. Bei der Kontrollgruppe (3 Zellkulturschalen) wurde für die Verdünnung des Zellkulturmediums anstelle des D20 die gleiche Menge H2O verwendet.

Die Bestimmung der Virusanzahl nach Beispiel 7 für Verum- und Kontrollkultur ergab Werte, die in Tabelle 1 dargestellt sind.

### Referenz-Beispiel 12: D20-Wirksamkeit bei Influenzavirus B 1 in Typ-1 Zellkultur

Es wurden 6 Zellkulturschalen der Zellkultur Typ 1 nach Beispiel 4 mit Influenzavirus B/Hongkong/5/72 infiziert. 120 Minuten nach der Infektion wurde das mit den Viren versetzte Zellkulturmedium entfernt und wie folgt durch ein verdünntes Zellkulturmedium ersetzt: Bei der Verumgruppe (3 Zellkulturschalen) durch 30% D20 und 70% DMEM mit 10% FCS und 100 U/ml Penicillin sowie 100 µg/ml Streptomycin. Bei der Kontrollgruppe (3 Zellkulturschalen) wurde für die Verdünnung des Zellkulturmediums anstelle des D20 die gleiche Menge H2O verwendet.

Die Bestimmung der Virusanzahl nach Beispiel 7 für Verum- und Kontrollkultur ergab Werte, die in Tabelle 1 dargestellt sind.

### Beispiel 13: D20-Wirksamkeit bei humanes Parainfluenzavirus 1 in Typ-1 Zellkultur

Es wurden 6 Zellkulturschalen der Zellkultur Typ 1 nach Beispiel 4 mit Humanen Parainfluenzaviren 1, Stamm Washington 1957 (HPIV-1) infiziert. 120 Minuten nach der Infektion wurde das mit den Viren versetzte Zellkulturmedium entfernt und wie folgt durch ein verdünntes Zellkulturmedium ersetzt: Bei der Verumgruppe (3 Zellkulturschalen) durch 30% D20 und 70% DMEM mit 10% FCS und 100 U/ml Penicillin sowie 100 µg/ml Streptomycin. Bei der Kontrollgruppe (3 Zellkulturschalen) wurde für die Verdünnung des Zellkulturmediums anstelle des D20 die gleiche Menge H2O verwendet.

Die Bestimmung der Virusanzahl nach Beispiel 7 für Verum- und Kontrollkultur ergab Werte, die in Tabelle 1 dargestellt sind.

### Beispiel 14: D20-Wirksamkeit bei humanes Respiratorisches Synzytialvirus (HRSV) 1 in Typ-1 Zellkultur

Es wurden 6 Zellkulturschalen der Zellkultur Typ 1 nach Beispiel 4 mit HSRV, Stamm Long, infiziert. 120 Minuten nach der Infektion wurde das mit den Viren versetzte Zellkulturmedium entfernt und wie folgt durch ein verdünntes Zellkulturmedium ersetzt: Bei der Verumgruppe (3 Zellkulturschalen) durch 30% D20 und 70% DMEM mit 10% FCS und 100 U/ml Penicillin sowie 100 µg/ml Streptomycin. Bei der Kontrollgruppe (3 Zellkulturschalen) wurde für die Verdünnung des Zellkulturmediums anstelle des D20 die gleiche Menge H2O verwendet.

Die Bestimmung der Virusanzahl nach Beispiel 7 für Verum- und Kontrollkultur ergab Werte, die in Tabelle 1 dargestellt sind.

### Beispiel 15: D2O-Wirksamkeit bei humanes Coxsackievirus 1 in Typ-1 Zellkultur

Es wurden 6 Zellkulturschalen der Zellkultur Typ 1 nach Beispiel 4 mit Humanen Coxsackieviren, Typ A1, infiziert. 120 Minuten nach der Infektion wurde das mit den Viren versetzte Zellkulturmedium entfernt und wie folgt durch ein verdünntes Zellkulturmedium ersetzt: Bei der Verumgruppe (3 Zellkulturschalen) durch 30% D20 und 70% DMEM mit 10% FCS und 100 U/ml Penicillin sowie 100 µg/ml Streptomycin. Bei der Kontrollgruppe (3 Zellkulturschalen) wurde für die Verdünnung des Zellkulturmediums anstelle des D20 die gleiche Menge H2O verwendet.

Die Bestimmung der Virusanzahl nach Beispiel 7 für Verum- und Kontrollkultur ergab Werte, die in Tabelle 1 dargestellt sind.

### Beispiel 16: D20-Wirksamkeit bei Humanen Adenoviren Typ 3 1 in Typ-1 Zellkultur

Es wurden 6 Zellkulturschalen der Zellkultur Typ 1 nach Beispiel 4 mit Humanen Adenoviren Typ 3, Stamm GZ1, infiziert. 120 Minuten nach der Infektion wurde das mit den Viren versetzte Zellkulturmedium entfernt und wie folgt durch ein verdünntes Zellkulturmedium ersetzt: Bei der Verumgruppe (3 Zellkulturschalen) durch 30% D20 und 70% DMEM mit 10% FCS und 100 U/ml Penicillin sowie 100 µg/ml Streptomycin. Bei der Kontrollgruppe (3 Zellkulturschalen) wurde für die Verdünnung des Zellkulturmediums anstelle des D20 die gleiche Menge H2O verwendet.

Die Bestimmung der Virusanzahl nach Beispiel 7 für Verum- und Kontrollkultur ergab Werte, die in Tabelle 1 dargestellt sind.

### Beispiel 17: D20-Wirksamkeit bei Humanen Enteroviren Typ EV71 1 in Typ-1 Zellkultur

Es wurden 6 Zellkulturschalen der Zellkultur Typ 1 nach Beispiel 4 mit Humanen Enteroviren Typ EV71, Stamm SHZH98, infiziert. 120 Minuten nach der Infektion wurde das mit den Viren versetzte Zellkulturmedium entfernt und wie folgt durch ein verdünntes Zellkulturmedium ersetzt: Bei der Verumgruppe (3 Zellkulturschalen) durch 30% D20 und 70% DMEM mit 10% FCS und 100 U/ml Penicillin sowie 100 µg/ml Streptomycin. Bei der Kontrollgruppe (3 Zellkulturschalen) wurde für die Verdünnung des Zellkulturmediums anstelle des D20 die gleiche Menge H2O verwendet.

Die Bestimmung der Virusanzahl nach Beispiel 7 für Verum- und Kontrollkultur ergab Werte, die in Tabelle 1 dargestellt sind.

### Beispiel 18: D20-Wirksamkeit bei Rhinoviren 1 in Typ-1 Zellkultur

Es wurden 6 Zellkulturschalen der Zellkultur Typ 1 nach Beispiel 4 mit Rhinoviren (Stamm RV-16 von American Type Culture Collection, Manassas, VA, USA) infiziert. 120 Minuten nach der Infektion wurde das mit den Viren versetzte Zellkulturmedium entfernt und wie folgt durch ein verdünntes Zellkulturmedium ersetzt: Bei der Verumgruppe (3 Zellkulturschalen) durch 30% D20 und 70% DMEM mit 10% FCS und 100 U/ml Penicillin sowie 100 µg/ml Streptomycin. Bei der Kontrollgruppe (3 Zellkulturschalen) wurde für die Verdünnung des Zellkulturmediums anstelle des D20 die gleiche Menge H2O verwendet.
Die Bestimmung der Virusanzahl nach Beispiel 7 für Verum- und Kontrollkultur ergab Werte, die in Tabelle 1 dargestellt sind.

### Beispiel 19: Präventive D20-Wirksamkeit bei Rhinoviren in modifizierter Typ-1 Zellkultur

Es wurden 6 Zellkulturschalen der modifizierten Zellkultur Typ 1 nach Beispiel 5 mit Rhinoviren (Stamm RV-16 von American Type Culture Collection Manassas, VA, USA), infiziert. 120 Minuten nach der Infektion wurde das mit den Viren versetzte Zellkulturmedium entfernt und wie folgt durch ein verdünntes Zellkulturmedium ersetzt: Bei der Verumgruppe (3 Zellkulturschalen) durch 30% D20 und 70% DMEM mit 10% FCS und 100 U/ml Penicillin sowie 100 µg/ml Streptomycin. Bei der Kontrollgruppe (3 Zellkulturschalen) wurde für die Verdünnung des Zellkulturmediums anstelle des D20 die gleiche Menge H2O verwendet. Die Bestimmung der Virusanzahl nach Beispiel 7 für Verum- und Kontrollkultur ergab Werte, die in Tabelle 2 dargestellt sind.

### Referenz-Beispiel 20: Präventive D20-Wirksamkeit bei Herpesviren Typ 2 in modifizierter Typ-1 Zellkultur

Es wurden 6 Zellkulturschalen der modifizierten Zellkultur Typ 1 nach Beispiel 5 mit Herpesviren Typ 2 (Stamm 734 von American Type Culture Collection, Manassas, VA, USA) infiziert. 120 Minuten nach der Infektion wurde das mit den Viren versetzte Zellkulturmedium entfernt und wie folgt durch ein verdünntes Zellkulturmedium ersetzt: Bei der Verumgruppe (3 Zellkulturschalen) durch 30% D20 und 70% DMEM mit 10% FCS und 100 U/ml Penicillin sowie 100 µg/ml Streptomycin. Bei der Kontrollgruppe (3 Zellkulturschalen) wurde für die Verdünnung des Zellkulturmediums anstelle des D20 die gleiche Menge H2O verwendet.

Die Bestimmung der Virusanzahl nach Beispiel 7 für Verum- und Kontrollkultur ergab Werte, die in Tabelle 2 dargestellt sind.

### Referenz-Beispiel 21: D20-Wirksamkeit bei Herpesviren HSV-1 in Typ-2 Zellkultur

Es wurden 6 Zellkulturschalen der Zellkultur Typ 2 nach Beispiel 6 mit Herpesviren vom Typ 1, HSV-1 (Stamm Maclntyre VR-539 von American Type Culture Collection, Manassas, VA, USA) infiziert. 120 Minuten nach der Infektion wurde das mit den Viren versetzte Zellkulturmedium entfernt und wie folgt durch ein verdünntes Zellkulturmedium ersetzt: Bei der Verumgruppe (3 Zellkulturschalen) durch 30% D20 und 70% DMEM mit 10% FCS und 100 U/ml Penicillin sowie 100 µg/ml Streptomycin. Bei der Kontrollgruppe (3 Zellkulturschalen) wurde für die Verdünnung des Zellkulturmediums anstelle des D20 die gleiche Menge H2O verwendet.

Die Bestimmung der Virusanzahl nach Beispiel 7 für Verum- und Kontrollkultur ergab Werte, die in Tabelle 3 dargestellt sind.

### Referenz-Beispiel 22: D20-Wirksamkeit bei Herpesviren Typ Varicella Zoster 1 in Typ-2 Zellkultur

Es wurden 6 Zellkulturschalen der Zellkultur Typ 2 nach Beispiel 6 mit Herpesviren vom Typ Zoster, Varicella Zoster, (Stamm Ellen, VR-586 von American Type Culture Collection, Manassas, VA, USA) infiziert. 120 Minuten nach der Infektion wurde das mit den Viren versetzte Zellkulturmedium entfernt und wie folgt durch ein verdünntes Zellkulturmedium ersetzt: Bei der Verumgruppe (3 Zellkulturschalen) durch 30% D20 und 70% DMEM mit 10% FCS und 100 U/ml Penicillin sowie 100 µg/ml Streptomycin. Bei der Kontrollgruppe (3 Zellkulturschalen) wurde für die Verdünnung des Zellkulturmediums anstelle des D20 die gleiche Menge H2O verwendet.

Die Bestimmung der Virusanzahl nach Beispiel 7 für Verum- und Kontrollkultur ergab Werte, die in Tabelle 3 dargestellt sind.

### Referenz-Beispiel 23: D20-Wirksamkeit bei Herpesviren HSV-2 in Typ-2 Zellkultur

Es wurden 6 Zellkulturschalen der modifizierten Zellkultur Typ 2 nach Beispiel 6 mit Herpesviren Typ 2 (Stamm 734 von American Type Culture Collection, Manassas, VA, USA) infiziert. 120 Minuten nach der Infektion wurde das mit den Viren versetzte Zellkulturmedium entfernt und wie folgt durch ein verdünntes Zellkulturmedium ersetzt: Bei der Verumgruppe (3 Zellkulturschalen) durch 30% D20 und 70% DMEM mit 10% FCS und 100 U/ml Penicillin sowie 100 µg/ml Streptomycin. Bei der Kontrollgruppe (3 Zellkulturschalen) wurde für die Verdünnung des Zellkulturmediums anstelle des D20 die gleiche Menge H2O verwendet. Die Bestimmung der Virusanzahl nach Beispiel 7 für Verum- und Kontrollkultur ergab Werte, die in Tabelle 3 dargestellt sind.

### Beispiel 24: D20-Wirksamkeit bei humanen Adenoviren in Typ-2 Zellkultur

Es wurden 6 Zellkulturschalen der Zellkultur Typ 2 nach Beispiel 6 mit Humanen Adenoviren Typ 3, Stamm GZ1, infiziert. 120 Minuten nach der Infektion wurde das mit den Viren versetzte Zellkulturmedium entfernt und wie folgt durch ein verdünntes Zellkulturmedium ersetzt: Bei der Verumgruppe (3 Zellkulturschalen) durch 30% D20 und 70% DMEM mit 10% FCS und 100 U/ml Penicillin sowie 100 µg/ml Streptomycin. Bei der Kontrollgruppe (3 Zellkulturschalen) wurde für die Verdünnung des Zellkulturmediums anstelle des D20 die gleiche Menge H2O verwendet.

Die Bestimmung der Virusanzahl nach Beispiel 7 für Verum- und Kontrollkultur ergab Werte, die in Tabelle 3 dargestellt sind.

### Beispiel 25: D20-Wirksamkeit von D20-Hydrogel und D2O-Augentropfen zur Therapie von Augenherpes im klinischen Versuch

Es wurden 33 gesunde Freiwillige im Alter von 20-65 Jahren (15 weiblich, 18 männlich) für diese Studie ausgewählt. Alle ausgewählten Testpersonen hatten eine Historie Herpes im Bereich des Auges, welcher überwiegend die Konjunktiva und in einigen Fällen auch die Cornea betraf, mit mindestens 5 Ereignissen pro Jahr.
Keine der Testpersonen benutzte eine Woche vor oder während des auf 3 Monate begrenzten Tests antivirale Therapeutika oder immunmodulierende Therapeutika.

Die 33 Testpersonen wurden randomisiert in 3 Gruppen (Verumgruppen A und B sowie Kontrolle) zu je 11 Personen aufgeteilt. Jede Testperson der Verumgruppe A und der Kontrolle erhielt eine 20 g Tube eines nach Beispiel 2 hergestellten Hydrogels, die Verumgruppe A ein D20-Hydrogel (hergestellt mit 100% D20), die Kontollgruppe ein H2O-Hydrogel (hergestellt mit 100% H2O) mit der Anweisung, dieses sofort bei ersten Symptomen eines Augenherpes auf der betroffenen Schleimhautstelle aufzutragen. Die Verumgruppe B erhielt ein 30 ml Flasche einer nach Beispiel 1 hergestellten D20-Augentropfen-Lösung. Die Verabreichung sollte für alle Gruppen alle 2 Stunden für die Dauer von 3 Tagen während der Wachphasen wiederholt werden. Die Verabreichungsmenge war ca. 0,5 ml des Hydrogels und 0,3 ml der Augentropfen. Die Testpersonen hatten die Anweisung, ein Protokoll der Verabreichung zu führen (Patienten-Tagebuch) und darin jede Verabreichung zu vermerken und weiterhin zu jeder Verabreichung folgende Symptomatik-Details (Symptom Score), bezogen auf den vorangegangenen Auftrag des Gels, auf einer Skala von 1 (sehr wenig/klein) bis 5 (sehr stark/groß) bewertet, einzutragen: a) Juckreiz b) Schmerzen, c) Trockenheit, d) Ausdehnung des betroffenen Bereichs der Konjunktiva. Diese 4 Größen wurden bei der Datenauswertung arithmetisch gemittelt und dieser Score anschließend über die Gesamtzahl der Einzelscores (=Anzahl der Verabreichungen) gemittelt. Weiterhin sollte Datum und Uhrzeit des Bemerkens der ersten Symptome, Zeitpunkt des Therapiebeginns, Zeitpunkt des äußerlich vollständigen Abheilens des Herpes, Zeitpunkt der Schmerzfreiheit sowie (soweit möglich) die Lage des Herpes angegeben werden. Für die Datenauswertung wurden nur Herpes-Ereignisse berücksichtigt, die sich im Konjunktivabereich befanden, und dort nur solche, bei denen die Testpersonen die Ausdehnung (mittels eines zur Verfügung gestellten Messstreifens vor dem Spiegel) ungefähr vermessen konnten. Der Zeitraum der Studie erstreckte sich über 3 Monate. Insgesamt wurden auf diese Weise verwertbare Daten von 7 Personen der Kontrollgruppe und 6 Personen der Verumgruppe A sowie 7 Personen der Verumgruppe B erhalten. Die Ergebnisse sind in Tabelle 4 dargestellt.

## Patentansprüche

1. Deuteriumoxid zur Verwendung für die Prophylaxe und/oder Therapie von Virus-basierten Erkrankungen des Auges, wobei es sich bei der Virus-basierten Erkrankung des Auges um Keratokonjunctivitis epidemica, Keratokonjunktivitis sicca, hemorrhagische Konjunktivitis, follikuläre Konjunktivitis, Pharyngokonjunktivalfieber, Blepharitis, Blepharokonjunktivitis, Keratitis dendritica, Keratitis interstitialis, Edotheliitis, Keratitis disciformis, Ophthalmia neonatorum, Dakryoadentitis, akute Dakryoadentitis, Dakryocystitis Episkleritis, Skleritis, Choroiditis Chorioretinitis Retinitis, akute nekrotisierende Retinitis, Uveitis anterior, Uveitis intermedia und/oder Uveitis posterior handelt, und wobei die Replikation der Virusnukleinsäure und damit die Virusvermehrung gehemmt oder inhibiert wird.

2. Deuteriumoxid zur Verwendung nach Anspruch 1, wobei Deuteriumoxid in einer Konzentration von mehr als 20% bezogen auf den Gesamtwasserhaushalt der Zelle verabreicht wird.

3. Deuteriumoxid zur Verwendung nach einem der vorangehenden Ansprüche, wobei es sich bei dem Virus um ein Virus der Familie Poxviridae, Paramyxoviridae, Coronaviridae, Chordopoxviridae, Togaviridae, Adenoviridae und/oder Picornaviridae handelt.

4. Deuteriumoxid zur Verwendung nach einem der vorangehenden Ansprüche, wobei es sich bei dem Virus um ein Virus der Gattung Cytomegalievirus, Chordopoxvirus, Rubivirus, Respirovirus,Rubulavirus, Adenovirus, Mastadenovirus, Orthopoxvirus, Enterovirus und/oder Rhinovirus handelt.

5. Deuteriumoxid zur Verwendung nach einem der vorangehenden Ansprüche, wobei es sich bei dem Virus um ein Virus der Art humanes Cytomgalievirus (HMZV), Epstein-Barr-Virus, Vacciniavirus, Rubivirus, Rötelvirus, Rubellavirus, Parainfluenzavirus 1, Parainfluenzavirus 2, Parainfluenzavirus 3, Parainfluenzavirus 4, Masernvirus, Mumpsvirus, humanes Adenovirus A, humanes Adenovirus B, humanes Adenovirus C, humanes Adenovirus D, humanes Adenovirus E, humanes Adenovirus F, Echovirus, Cosackievirus A, Cosackievirus B, Rhinovirus, Tollwutvirus, Respiratorisches Synzytialvirus und/oder Rifttal-Fieber-Virus handelt.

6. Deuteriumoxid zur Verwendung nach einem der vorangehenden Ansprüche, wobei das Deuteriumoxid topisch verabreicht wird.

7. Deuteriumoxid zur Verwendung nach einem der vorangehenden Ansprüche, wobei das Deuteriumoxid das Auge, insbesondere die Schleimhäute des Auges, hydratisiert.

8. Deuteriumoxid zur Verwendung nach einem der vorangehenden Ansprüche, wobei das D20 in Kombination mit mindestens einem weiteren pharmazeutischen Wirkstoff und/oder mindestens einem weiteren nicht-pharmazeutischen Wirkstoff verwendet wird.

9. Deuteriumoxid zur Verwendung nach Anspruch 8, wobei der mindestens eine weitere pharmazeutische Wirkstoff ausgewählt ist aus der Gruppe bestehend aus Virostatika, Proteinen, Peptiden, Nukleinsäuren und immunosuppressiven Wirkstoffen.

10. Deuteriumoxid zur Verwendung nach Anspruch 8, wobei der mindestens eine weitere nicht-pharmazeutische Wirkstoff ausgewählt ist aus der Gruppe bestehend aus pharmazeutisch verträglichen anorganischen oder organischen Säuren oder Basen, Salzen, Vitaminen, Provitaminen, Polymeren, Copolymeren, Block-Copolymeren, Einfachzucker, Mehrfachzucker, ionischen und nicht-ionischen Tensiden oder Lipiden sowie Mischungen hiervon, Albumin, Transferrin und DNA-Reparatur-Proteinen, wie Kinase-Inhibitoren.

11. Deuteriumoxid zur Verwendung nach einem beliebigen der vorangegangenen Ansprüche, wobei D20 als Formulierung verabreicht wird, und wobei die Formulierung vorzugsweise eine Flüssigkeit, Creme, Salbe, Gel oder ein Hydrogel ist.

## Claims

1. A deuterium oxide for use for prevention and/or treatment of viral diseases of the eye, wherein the viral disease of the eye is epidemic keratoconjunctivitis, keratoconjunctivitis sicca, hemorrhagic conjunctivitis, follicular conjunctivitis, pharyngoconjunctival fever, blepharitis, blepharoconjunctivitis, keratitis dendritica, interstitial keratitis, endotheliitis, keratitis disciformis, ophthalmia neonatorum, dacryoadenitis, acute dacryoadenitis, dacryocystitis, episcleritis, scleritis, choroiditis, chorioretinitis, retinitis, acute necrotizing retinitis, anterior uveitis, intermediate uveitis and/or posterior uveitis, and wherein the replication of the viral nucleic acid and thus the replication of the virus is inhibited or suppressed.

2. The deuterium oxide for use according to Claim 1, wherein deuterium oxide is administered in a concentration of more than 20%, based on the total water balance of the cell.

3. The deuterium oxide for use according to any one of the preceding claims, wherein the virus is a virus of the family Poxviridae, Paramyxoviridae, Coronaviridae, Chordopoxviridae, Togaviridae, Adenoviridae and/or Picornaviridae.

4. The deuterium oxide for use according to any one of the preceding claims, wherein the virus is a virus of the genus cytomegalovirus, chordopoxvirus, rubivirus, respirovirus, rubulavirus, adenovirus, mastadenovirus, orthopoxvirus, enterovirus and/or rhinovirus.

5. The deuterium oxide for use according to any one of the preceding claims, wherein the virus is a virus of the species human cytomegalovirus (HCMV), Epstein-Barr virus, vaccinia virus, rubivirus, German measles virus, rubella virus, parainfluenza virus 1, parainfluenza bvirus 2, parainfluenza virus 3, parainfluenza virus 4, measles virus, mumps virus, human adenovirus A, human adenovirus B, human adenovirus C, human adenovirus D, human adenovirus E, human adenovirus F, echovirus, cosackievirus A, cosackievirus B, rhinovirus, rabies virus, respiratory syncytial virus and/or Rift Valley fever virus.

6. The deuterium oxide for use according to any one of the preceding claims, wherein the deuterium oxide is administered topically.

7. The deuterium oxide for use according to any one of the preceding claims, wherein the deuterium oxide hydrates the eye, in particular the mucous membranes of the eye.

8. The deuterium oxide for use according to any one of the preceding claims, wherein the D20 is used in combination with at least one additional pharmaceutical active ingredient and/or at least one additional non-pharmaceutical active ingredient.

9. The deuterium oxide for use according to Claim 8, wherein the at least one additional pharmaceutical active ingredient is selected from the group consisting of virostatics, proteins, peptides, nucleic acids and immunosuppressant active ingredients.

10. The deuterium oxide for use according to Claim 8, wherein the at least one additional non-pharmaceutical active ingredient is selected from the group consisting of pharmaceutically acceptable organic or inorganic acids or bases, salts, vitamins, provitamins, polymers, copolymers, block copolymers, monosaccharides, polysaccharides, ionic and nonionic surfactants or lipids as well as mixtures thereof, albumin, transferrin and DNA repair proteins such as kinase inhibitors.

11. The deuterium oxide for use according to any one of the preceding claims, wherein D20 is administered as a formulation and wherein the formulation is preferably a liquid, cream, ointment, gel or hydrogel.

## Revendications

1. Oxyde de deutérium pour son utilisation pour la prophylaxie et/ou le traitement d'affections d'origine virale de l'oeil, caractérisé en ce quel'affection d'origine virale de l'oeil est la kératoconjonctivite épidémique, la kératoconjonctivite sèche, la conjonctivite hémorragique, la conjonctivite folliculaire, la fièvre pharyngo-conjonctivale, la blépharite, le blépharoconjonctivite, la kératite dendritique, la kératite interstitielle, l'endothélite, la kératite disciforme, l'ophtalmie néonatale, la dacryoadénite, la dacryoadénite aiguë, la dacryocystite, l'épisclérite, la sclérite, la choroïdite, la choriorétinite, la rétinite, la rétinite nécrosante aiguë, l'uvéite antérieure, l'uvéite intermédiaire et/ou l'uvéite postérieure, et **caractérisé en ce que** la réplication de l'acide nucléique viral et, de ce fait la multiplication virale, est bloquée ou inhibée.

2. Oxyde de deutérium pour son utilisation selon la revendication 1, **caractérisé en ce que** l'oxyde de deutérium est administré à une concentration supérieure à 20 % par rapport à l'équilibre hydrique total de la cellule.

3. Oxyde de deutérium pour son utilisation selon l'une des revendications précédentes, **caractérisé en ce que** le virus est un virus de la famille des poxvirus, des paramyxovirus, des coronavirus, des chordopoxvirus, des togavirus, des adénovirus et/ou des picornavirus.

4. Oxyde de deutérium pour son utilisation selon l'une des revendications précédentes, **caractérisé en ce que** le virus est un virus du genre cytomégalovirus, chordopoxvirus, rubivirus, respirovirus, rubulavirus, adénovirus, mastadénovirus, orthopoxvirus, entérovirus et/ou rhinovirus.

5. Oxyde de deutérium pour son utilisation selon l'une des revendications précédentes, **caractérisé en ce que** le virus est un virus de l'espèce cytomégalovirus humain (HCMV), virus d'Epstein-Barr, virus de la vaccine, rubivirus, virus de la rubéole, rubellavirus, virus para-influenza 1, virus para-influenza 2, virus para-influenza 3, virus para-influenza 4, virus de la rougeole, virus des oreillons, adénovirus A humain, adénovirus B humain, adénovirus C humain, adénovirus D humain, adénovirus E humain, adénovirus F humain, échovirus, coxsachievirus A, coxsachievirus B, rhinovirus, virus de la rage, virus syncytial respiratoire et/ou virus de la fièvre de la vallée du Rift.

6. Oxyde de deutérium pour son utilisation selon l'une des revendications précédentes, **caractérisé en ce que** l'oxyde de deutérium est administré par voie topique.

7. Oxyde de deutérium pour son utilisation selon l'une des revendications précédentes, **caractérisé en ce que** l'oxyde de deutérium hydrate l'oeil, en particulier, la muqueuse de l'oeil.

8. Oxyde de deutérium pour son utilisation selon l'une des revendications précédentes, **caractérisé en ce que** le D20 est utilisé en combinaison avec au moins une autre substance pharmaceutiquement active et/ou au moins une autre substance active non pharmaceutique.

9. Oxyde de deutérium pour son utilisation selon la revendication 8, **caractérisé en ce que** l'au moins une autre substance active pharmaceutique est choisie dans le groupe constitué de virostatiques, protéines, peptides, acides nucléiques et substances actives immunosuppressives.

10. Oxyde de deutérium, pour son utilisation selon la revendication 8,**caractérisé en ce que** l'au moins une autre substance active non pharmaceutique est choisie dans le groupe constitué d'acides ou bases inorganiques ou organiques, de sels, vitamines, provitamines, polymères, copolymères, copolymères à blocs, sucres simples, sucres complexes, tensioactifs ioniques et non ioniques ou lipides, pharmaceutiquement acceptables, et leurs mélanges, d'albumine, de transferrine et de protéines réparatrices de l'ADN telles qu'inhibiteurs de kinases.

11. Oxyde de deutérium pour son utilisation selon l'une quelconque des revendications précédentes, **caractérisé en ce que** le D20 est administré sous la forme d'une formulation et la formulation est de préférence un fluide, une crème, une pommade, un gel ou un hydrogel.
